# EUROPÄISCHE PATENTSCHRIFT

(11) **EP 0 847 432 B1**
(45) Veröffentlichungstag und Bekanntmachung des Hinweises auf die Patenterteilung: **27.06.2001**
(21) Anmeldenummer: 96930103.5
(22) Anmeldetag: 26.08.1996
(51) Int. Cl.: C09K 19/20, C09K 19/38, C07C 69/96, C09D 5/00

(54) **POLYMERISIERBARE FLÜSSIGKRISTALLINE VERBINDUNGEN**
POLYMERIZABLE LIQUID-CRYSTALLINE COMPOUNDS
COMPOSES DE CRISTAUX LIQUIDES POLYMERISABLES

(30) Priorität: 01.09.1995 DE 19532408
(43) Veröffentlichungstag der Anmeldung: 17.06.1998
(73) Patentinhaber: BASF AKTIENGESELLSCHAFT, 67056 Ludwigshafen (DE)
(72) Erfinder: MEYER, Frank, Dr., 68165 Mannheim (DE); SIEMENSMEYER, Karl, D-67227 Frankenthal (DE); ETZBACH, Karl-Heinz, D-67227 Frankenthal (DE); SCHUHMACHER, Peter, D-68163 Mannheim (DE)
(86) Internationale Anmeldenummer: EP9603756
(87) Internationale Veröffentlichungsnummer: WO9700600

(56) Entgegenhaltungen:
- EP-A- 0 643 121
- WO-A-94/08268
- DE-A- 4 226 994
- DE-A- 4 405 316
- DE-A- 4 408 170
- FR-A- 2 609 999

## Beschreibung

Die vorliegende Erfindung betrifft neue polymerisierbare flüssigkristalline Verbindungen der allgemeinen Formel I

Z¹-Y¹-A¹-Y³-M-Y⁴-A²-Y²-Z² I

in der die Variablen folgende Bedeutung haben:
- Z¹, Z²: Reste mit reaktiven Gruppen, über die eine Polymerisation herbeigeführt werden kann,
- Y¹ - Y⁴: chemische Einfachbindung, Sauerstoff, Schwefel, -O-CO-, -CO-O-, O-CO-O-, -CO-NR-, -NR-CO-, -O-CO-NR-, -NR-CO-O- oder -NR-CO-NR, wobei mindestens eine der Gruppen Y³ und Y⁴ -O-CO-O-, -O-CO-NR-, -NR-CO-O- oder -NR-CO-NR- bedeutet.
- A¹, A²: Spacer mit 2 bis 30 C-Atomen, in welchen die Kohlenstoffkette durch Sauerstoff in Etherfunktion, Schwefel in Thioetherfunktion oder durch nichtbenachbarte Imino- oder C₁-C₄-Alkyliminogruppen unterbrochen sein kann,
- M: eine mesogene Gruppe,
- R: C₁-C₄-Alkyl.

Weiterhin betrifft die Erfindung Flüssigkristallzusammensetzungen, welche diese Verbindungen und ggf. eine oder mehrere chirale Verbindungen enthalten, Verfahren zur Herstellung der erfindungsgemäßen flüssigkristallinen Verbindungen, Verfahren zur Beschichtung von Substraten mit den erfindungsgemäßen Verbindungen oder Flüssigkristallzusammensetzungen sowie die Verwendung der Verbindungen oder Flüssigkristallzusammensetzungen für die Herstellung optischer Anzeigegeräte, als cholesterisch flüssigkristalline Farbmittel sowie Pigmente, erhältlich durch Polymerisation der Flüssigkristallzusammensetzungen und anschließendes Zerkleinern.

Zahlreiche Verbindungen gehen beim Erwärmen vom kristallinen Zustand mit definierter Nah- und Fernordnung der Moleküle nicht direkt in den flüssigen, ungeordneten Zustand über, sondern durchlaufen dabei eine flüssigkristalline Phase, in welcher die Moleküle zwar beweglich sind, die Molekülachsen jedoch eine geordnete Struktur ausbilden. Gestreckte Moleküle bilden oft nematische flüssigkristalline Phasen, die durch eine Orientierungsfernordnung durch Parallellagerung der Moleküllängsachsen gekennzeichnet sind. Enthält eine derartige nematische Phase chirale Verbindungen, entsteht eine sogenannte cholesterische Phase, welche durch eine helixartige Überstruktur der Moleküllängsachsen gekennzeichnet ist. Die chirale Verbindung kann dabei die flüssigkristalline Verbindung selbst sein, oder sie kann als chiraler Dotierstoff einer nematischen flüssigkristallinen Phase zugesetzt sein.

Flüssigkristalline Materialien weisen bemerkenswerte optische Eigenschaften auf, die auf ihrem anisotropen Ordnungszustand beruhen. Der flüssigkristalline Ordnungszustand tritt jedoch nur in einem begrenzten Temperaturbereich auf. Oft liegt der Temperaturbereich, in dem flüssigkristalline Phasen auftreten, weit oberhalb der gewünschten Anwendungstemperatur oder er erstreckt sich nur über ein kleines Temperaturintervall.

Es gibt verschiedene Möglichkeiten, die für die Materialeigenschaften erwünschten Ordnungsstrukturen auch im festen Zustand zu erhalten und zu fixieren. Neben glasartigem Erstarren beim Abkühlen aus dem flüssigkristallinen Zustand besteht die Möglichkeit des Einpolymerisierens in polymere Netzwerke oder, für den Fall, daß die flüssigkristallinen Verbindungen polymerisierbare Gruppen enthalten, der Polymerisation der flüssigkristallinen Verbindungen selbst.

Polymerisierbare flüssigkristalline Verbindungen sind beispielsweise in der EP-A 261 712 und den Schriften WO 93/05436, WO 95/24453, WO 95/24454 und WO 95/24455 beschrieben. Diese Verbindungen weisen in polymerer Form meist die erforderlichen mechanischen Stabilitäten auf, lassen jedoch hinsichtlich der Temperaturhöhe ihrer flüssigkristallinen Phasen sowie der Temperaturbreite dieser Phasen teilweise zu wünschen übrig.

Aufgabe der vorliegenden Erfindung war es daher, polymerisierbare flüssigkristalline Verbindungen oder Flüssigkristallzusammensetzungen bereitzustellen, welche niedrige flüssigkristalline Phasentemperaturen, breite flüssigkristalline Phasenbereiche sowie, im polymeren Zustand, eine gute mechanische Festigkeit und Fixierung des flüssigkristallinen Ordnungszustandes aufweisen.

Demgemäß wurden die eingangs beschriebenen polymerisierbaren flüssigkristallinen Verbindungen und diese enthaltende Flüssigkristallzusammensetzungen gefunden.

Unter Polymerisation werden hierbei alle Aufbaureaktionen von Polymeren verstanden, also Additionspolymerisationen als Kettenreaktionen, Additionspolymerisationen als Stufenreaktionen sowie Kondensationspolymerisationen.

Bevorzugte Reste Z¹ und Z² sind
H₂C = CH ―, HC≡C―, 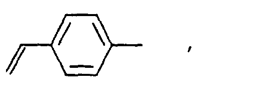 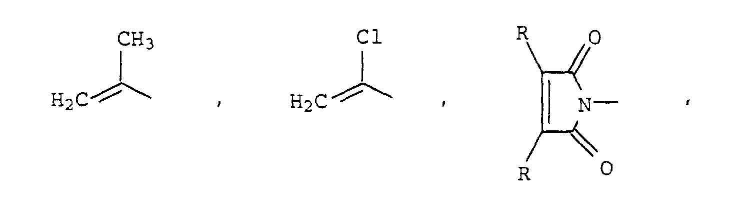 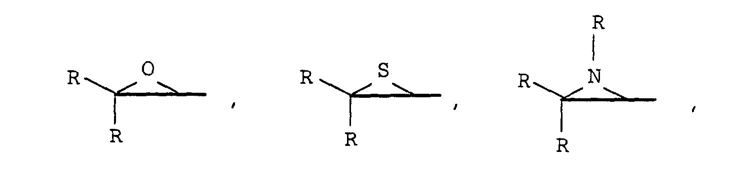 ― N = C = O, ― N = C = S und ― O ― C ≡ N,
wobei die Reste R für C₁-C₄-Alkyl stehen und gleich oder verschieden sein können.

Von den reaktiven polymerisierbaren Gruppen können die Cyanate spontan zu Cyanuraten trimerisieren und sind daher bevorzugt zu nennen. Verbindungen mit Epoxid-, Thiiran-, Aziridin-, Isocyanat- und Isothiocyanatgruppen benötigen zur Polymerisation weitere Verbindungen mit komplementären reaktiven Gruppen. So können beispielsweise Isocyanate mit Alkoholen zu Urethanen und mit Aminen zu Harnstoffderivaten polymerisieren. Analoges gilt für Thiirane und Aziridine. Die komplementären reaktiven Gruppen können dabei entweder in einer zweiten erfindungsgemäßen Verbindung vorhanden sein, die mit der ersteren gemischt wird, oder sie können durch Hilfsverbindungen, die zwei oder mehr dieser komplementären Gruppen enthalten, in das Polymerisationsgemisch eingebracht werden. Enthalten diese Verbindungen jeweils zwei dieser reaktiven Gruppen, so entstehen lineare Polymere mit überwiegend thermoplastischem Charakter. Enthalten die Verbindungen mehr als zwei reaktive Gruppen, so entstehen vernetzte Polymere, die mechanisch besonders stabil sind. Die Maleinimidogruppe eignet sich besonders zur radikalischen Copolymerisation mit olefinischen Verbindungen wie Styrol.

Bevorzugte polymerisierbare Gruppen Z¹ und Z² sind diejenigen, die der radikalischen Polymerisation zugänglich sind, also besonders die olefinisch ungesättigten Gruppen, und unter diesen haben in Kombination mit Y¹ bzw. Y² die Gruppen 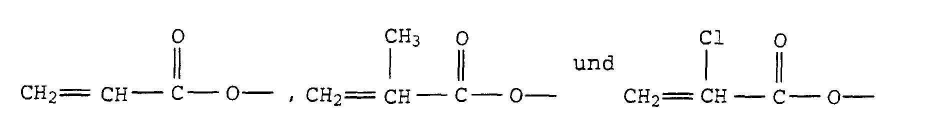 besondere Bedeutung.

Die in den erfindungsgemäßen Verbindungen enthaltenen Molekülteile Z¹, Z², A¹, A², M und X sind über Brückenglieder Y¹ - Y⁴ wie -O-, -S-, -CO-O-, -O-CO-, -O-CO-O-, -CO-NR-, -NR-CO-, -O-CO-NR-, -NR-CO-O-, -NR-CO-NR- oder auch über eine direkte Bindung miteinander verknüpft, wobei mindestens eine Verknüpfung des Spacers A¹ bzw. A² mit der mesogenen Gruppe durch eine Carbonatgruppe (-OCOO-), eine Carbamatgruppe (-O-CO-NR- oder -NR-CO-O-) oder eine Harnstoffgruppe (-NR-CO-NR-) erfolgt. Chirale polymerisierbare Verbindungen mit einer dieser Gruppen haben die vorteilhafte Eigenschaft besonders niedriger Phasenumwandlungstemperaturen und breiter Phasenbereiche und sind somit für Anwendungen bei Raumtemperatur besonders geeignet. Dies trifft besonders für die Carbonatgruppe zu.

Als Spacer A¹ und A² kommen alle für diesen Zweck bekannten Gruppen in Betracht. Die Spacer enthalten in der Regel 2 bis 30, vorzugsweise 3 bis 12 C-Atome und bestehen aus vorwiegend linearen aliphatischen Gruppen. Sie können in der Kette z.B. durch nicht benachbarte Sauerstoff- oder Schwefelatome oder Imino- oder Alkyliminogruppen wie Methyliminogruppen unterbrochen sein. Als Substituenten für die Spacerkette kommen dabei noch Fluor, Chlor, Brom, Cyan, Methyl und Ethyl in Betracht.

Repräsentative Spacer sind beispielsweise:
-(CH₂)ₚ-, -(CH₂CH₂O)ₘCH₂CH₂-, -CH₂CH₂SCH₂CH₂-, -CH₂CH₂NHCH₂CH₂-, 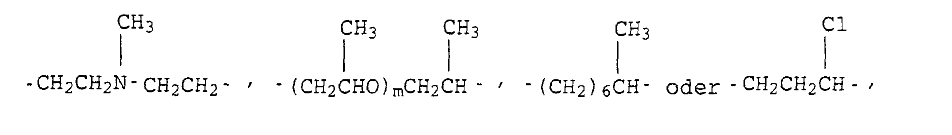 wobei
m für 1 bis 3 und p für 1 bis 12 steht.

Als Reste M können alle bekannten mesogenen Gruppen dienen. Insbesondere kommen Gruppen der Formel Ia

-(-T-Y⁵-)ᵣ-T- Ia

in Betracht, in der die Variablen folgende Bedeutung haben:
- T: zweiwertige gesättigte oder ungesättigte iso- oder heterocyclische Reste,
- Y⁵: Brückenglieder gemäß der Definition für Y¹ - Y⁴; -CH₂-O-; -O-CH₂-; -CH=N-, -N=CH- oder -N=N-,
- r: 0, 1, 2 oder 3,
wobei die Reste T und Y⁵ im Falle r > O gleich oder verschieden sein können.

Vorzugsweise ist r gleich 1 oder 2.

Die Reste T können auch durch Fluor, Chlor, Brom, Cyan, Hydroxy oder Nitro substituierte Ringsysteme sein. Bevorzugte Reste T sind:

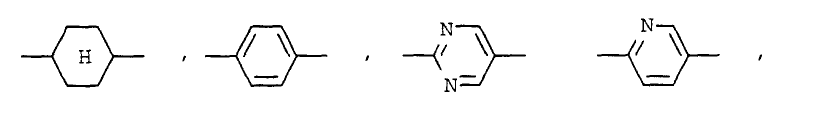

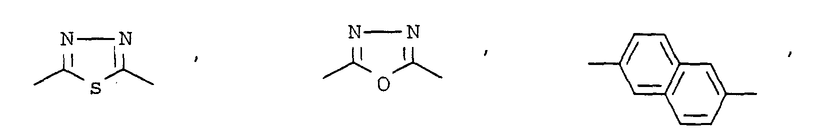

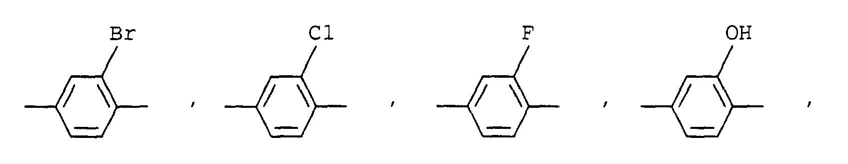

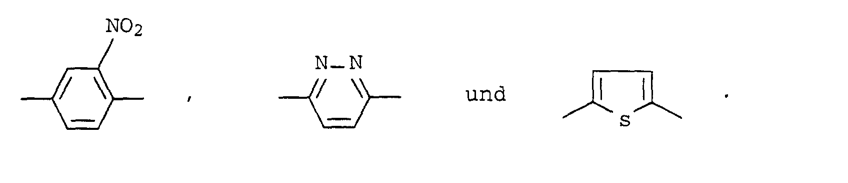

Bevorzugt als mesogene Gruppen M sind z.B.: 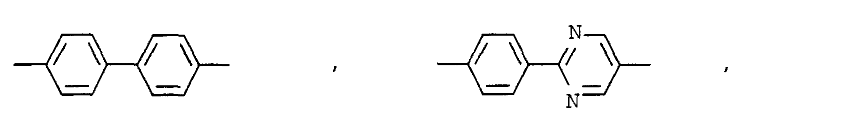 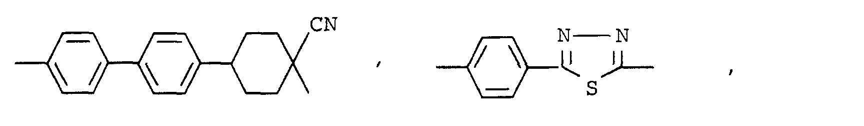 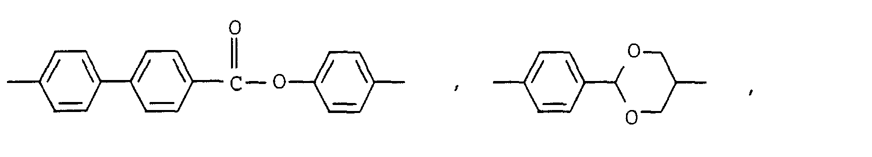 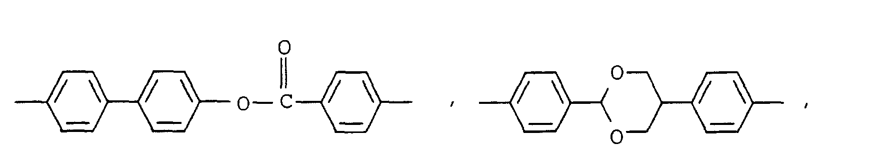 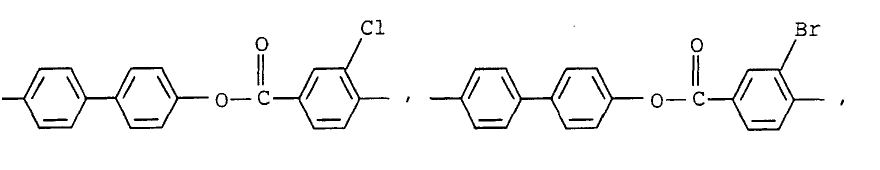 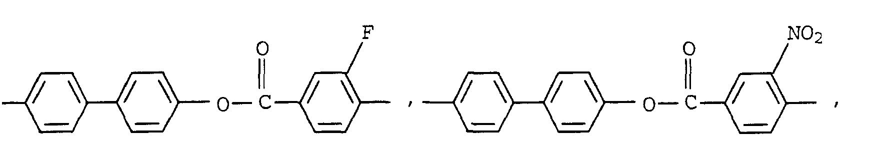 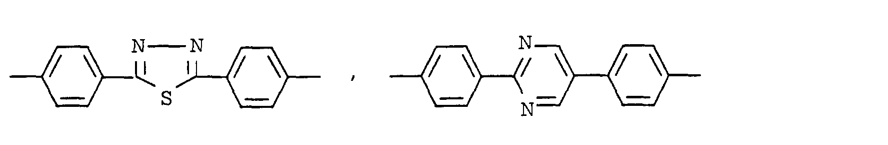 oder

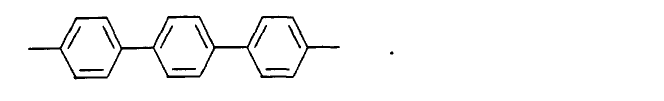

Besonders bevorzugt sind mesogene Gruppen M der folgenden Formeln 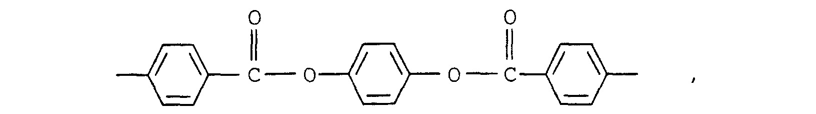 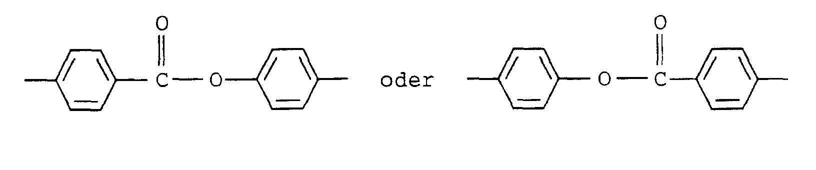 wobei jeder Ring bis zu drei gleiche oder verschiedene Substituenten aus der folgenden Gruppe tragen kann:

C₁-C₂₀-Alkyl, C₁-C₂₀-Alkoxy, C₁-C₂₀-Alkoxycarbonyl, C₁-C₂₀-Monoalkylaminocarbonyl, C₁-C₂₀-Alkylcarbonyl, C₁-C₂₀-Alkylcarbonyloxy, C₁-C₂₀-Alkylcarbonylamino, Formyl, Halogen, Cyan, Hydroxy oder Nitro.

Bevorzugte Substituenten für die aromatischen Ringe sind neben Fluor, Chlor, Brom, Cyan, Formyl und Hydroxy vor allem kurzkettige aliphatische Reste wie Methyl, Ethyl, n-Propyl, Isopropyl, n-Butyl, Isobutyl, tert.-Butyl sowie Alkoxy-, Alkoxycarbonyl-, Alkylcarbonyl-, Alkylcarbonyloxy-, Alkylcarbonylamino- und Monoalkylaminocarbonylreste, die diese Alkylgruppen enthalten.

Die äußeren Benzolringe der besonders bevorzugten Gruppen M haben vorzugsweise folgende Substitutionsmuster: 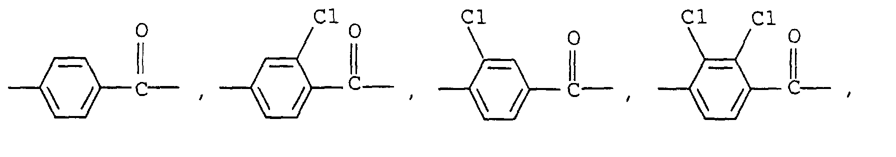 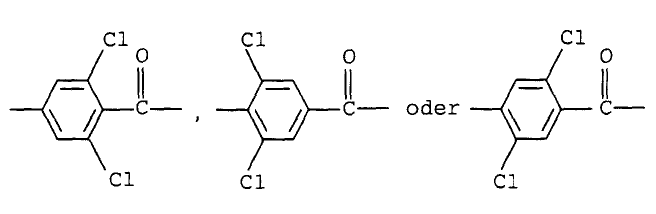 oder sie sind analog mit F, Br, CH₃, OCH₃, CHO, COCH₃, OCOCH₃ oder CN anstelle von Cl substituiert, wobei die Substituenten auch gemischt vorliegen können. Ferner sind die Strukturen 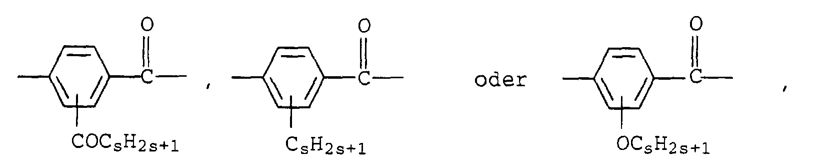 zu nennen, bei denen s 2 bis 20, vorzugsweise 8 bis 15, bedeutet.

Die bevorzugten Substitutionsmuster des mittleren Benzolrings der besonders bevorzugten Gruppen M sind 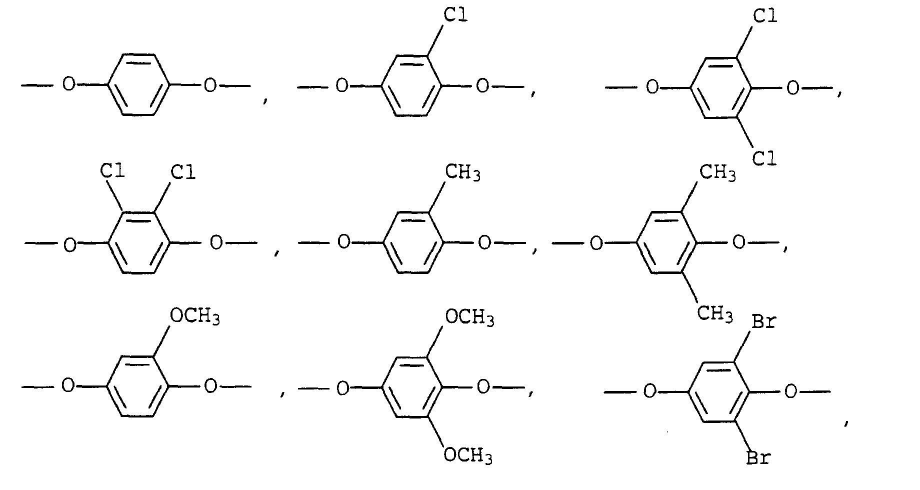 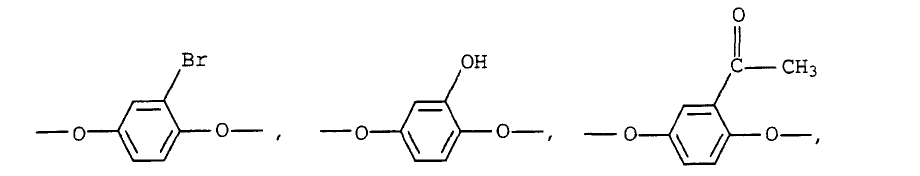 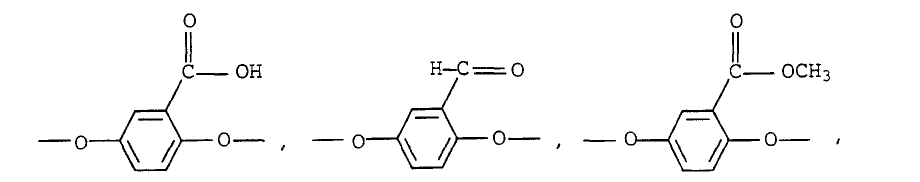 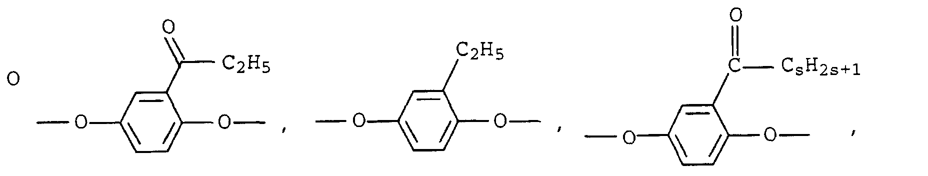 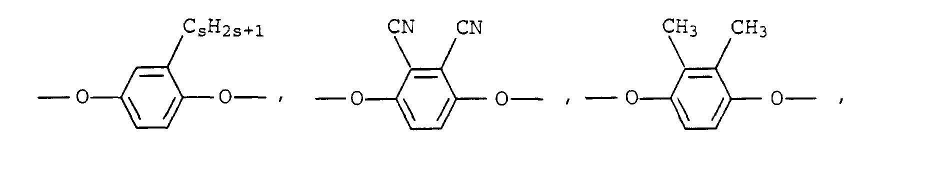 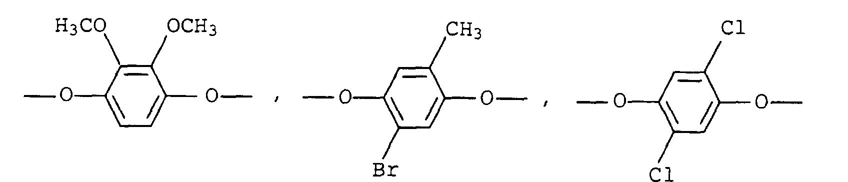 oder

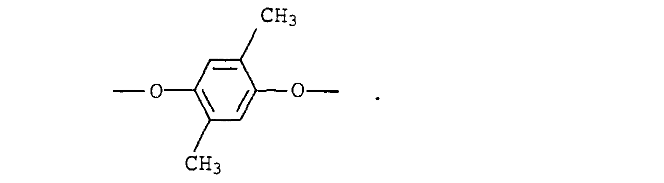

Bevorzugte erfindungsgemäße Verbindungen I sind auch solche, in denen die Restepaare Z¹ und Z², Y¹ und Y², Y³ und Y⁴ sowie A¹ und A² jeweils gleich sind.

Für technische Anwendungen, beispielsweise im Druckbereich, ist oft die Einstellung einer gewünschten Viskosität von Bedeutung.

Von den erfindungsgemäßen Verbindungen I können daher u.a. zu diesem Zweck auch Mischungen hergestellt werden. Solche Mischungen besitzen meist eine gegenüber den reinen Mischungskomponenten reduzierte Viskosität und haben in der Regel niedrigere flüssigkristalline Phasentemperaturen, so daß sie teilweise für Anwendungen bei Raumtemperatur geeignet sind.

In den Mischungen der erfindungsgemäßen Verbindungen können nicht nur z.B. "dreikernige", gegebenenfalls an den Ringen substituierte, mesogene Gruppen M der Formel 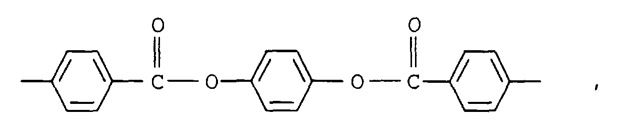 hierbei bedeuten in Formel Ia
- Y⁵: 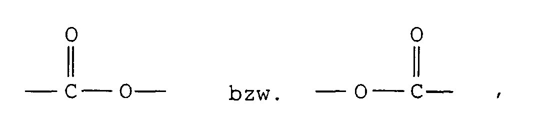
- T: drei gleiche Reste 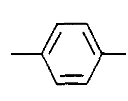 - (für den unsubstituierten Fall),
- r: gleich 2,
als Molekülfragmente auftreten, sondern z.B. auch "Zweikernringe" Gruppen M der Formeln 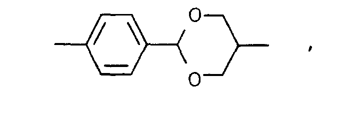 worin in Formel Ia zu setzen ist für
- Y⁵: eine chemische Einfachbindung,
- T: unterschiedliche Reste 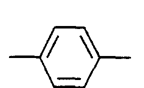 (ungesättigt isocyclisch) und 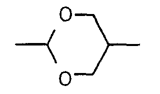 (gesättigt heterocyclisch),
- r: gleich 1,
oder 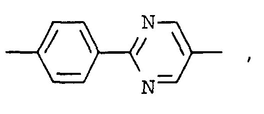 worin in Formel Ia bedeuten
- Y⁵: eine chemische Einfachbindung,
- T: unterschiedliche Reste 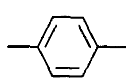 - (ungesättigt isocyclisch)
und 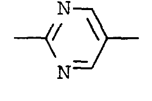 (ungesättigt heterocyclisch),
- r: gleich 1.

Besonders bevorzugte "zweikernige" mesogene Gruppen M sind dabei die Fragmente 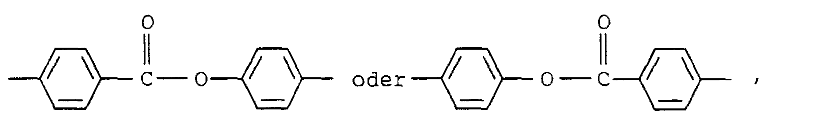 welche zusätzlich noch an den aromatischen Ringen, wie vorstehend beschrieben, substituiert sein können.

Weiter werden Flüssigkristallzusammensetzungen erfindungsgemäß beansprucht, welche neben Verbindungen I eine oder mehrere Verbindungen der allgemeinen Formel II

Z³-Y⁶-A³-Y⁷-M-Y⁸-P¹ II,

in der die Variablen folgende Bedeutung haben:
- Z³: Reste mit reaktiven Gruppen, über die eine Polymerisation herbeigeführt werden kann,
- Y⁶: Sauerstoff, Schwefel, -O-CO-, -CO-O-, -O-CO-O-, -CO-NR-, -NR-CO-, -O-CO-NR-, -NR-CO-O- oder -NR-CO-NR-,
- Y⁷, Y⁸: chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y⁷ und Y⁸ -O-CO-O-, -O-CO-NR-, -NR-CO-O oder -NR-CO-NR- bedeutet,
- A³: Spacer mit 2 bis 30 C-Atomen, in welchen die Kohlenstoffkette durch Sauerstoff in Etherfunktion, Schwefel in Thioetherfunktion oder durch nichtbenachbarte Imino- oder C₁-C₄-Alkyliminogruppen unterbrochen sein kann,
- P¹: Reste, ausgewählt aus der Gruppe Wasserstoff, C₁-C₃₀-Alkyl, C₁-C₃₀-Acyl, C₃-C₈-Cycloalkyl ggf. substituiert durch ein bis drei C₁-C₆-Alkyl und wobei die Kohlenstoffkette der Alkyl-, Acyl- und Cycloalkylreste durch Sauerstoff in Etherfunktion, Schwefel in Thioetherfunktion oder durch nichtbenachbarte Imino- oder C₁-C₄-Alkyliminogruppen unterbrochen sein kann,
enthalten können.Beispiele solcher ebenfalls beanspruchten Verbindungen der Formel II sind:

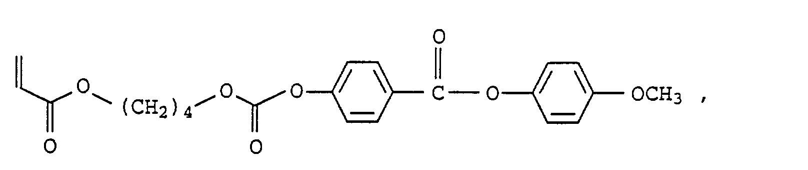

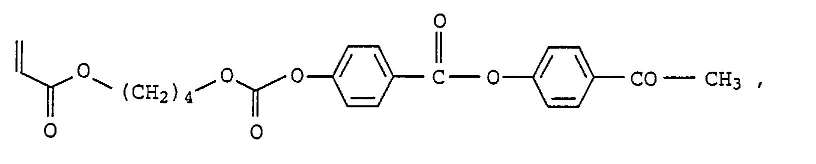

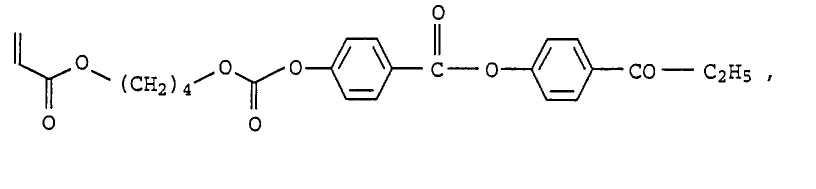

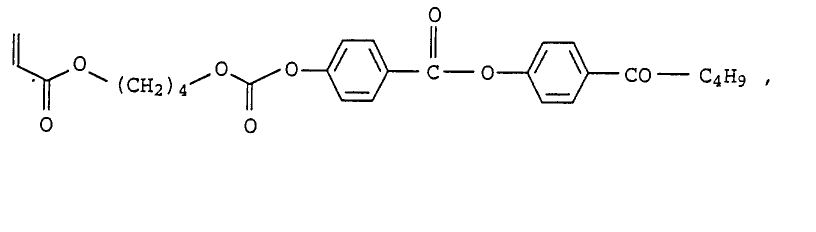

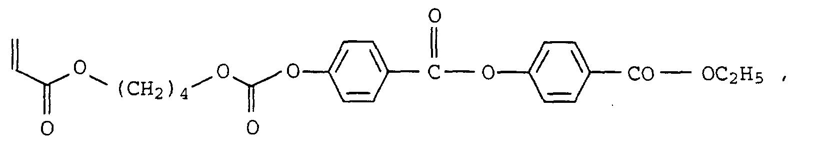

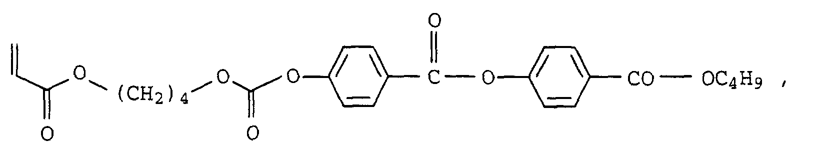

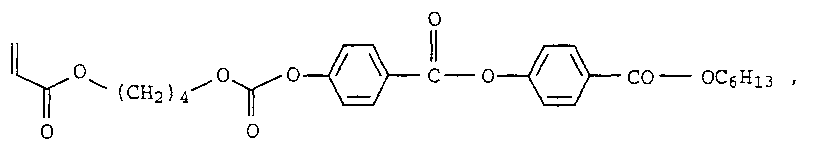

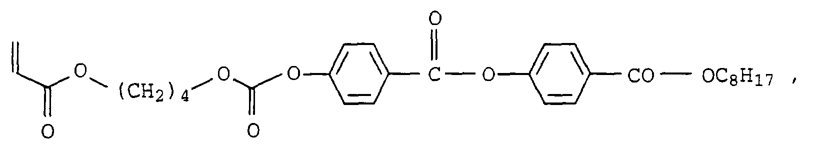

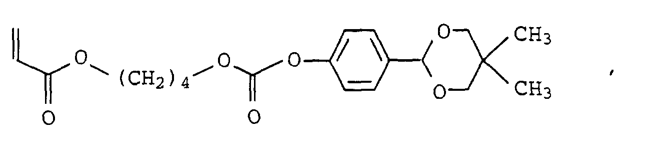

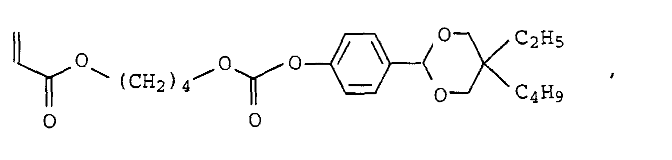

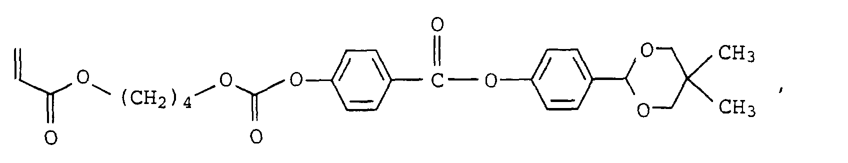

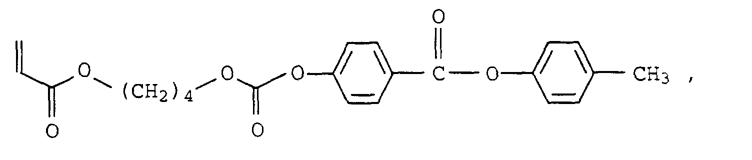

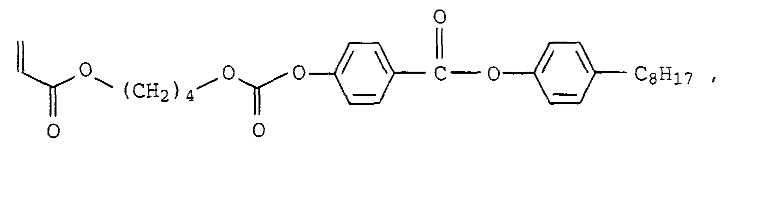

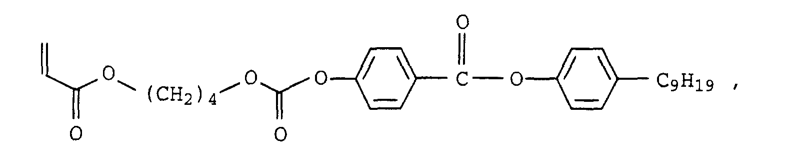

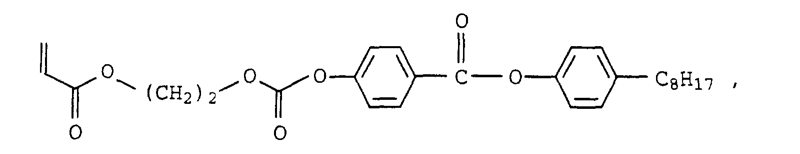

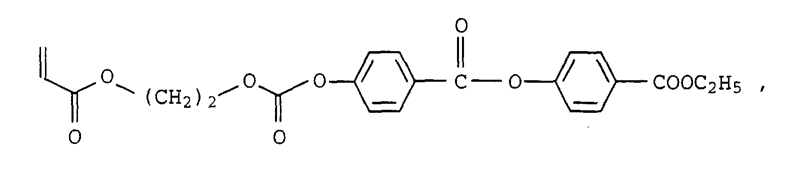

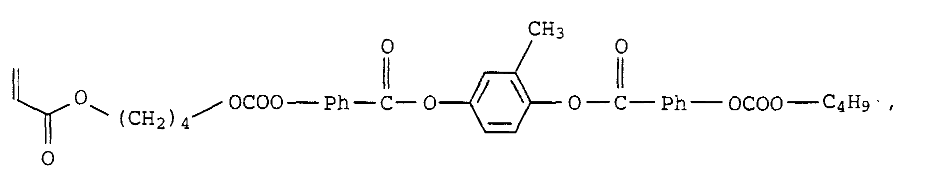

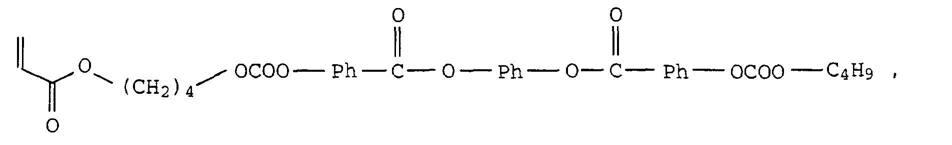

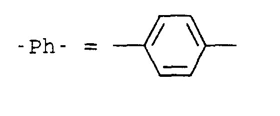

Weiter können noch eine oder mehrere Verbindungen der Formel III zugesetzt werden:

P²-Y⁹-M-Y¹⁰-P³ III,

in der die Variablen folgende Bedeutung haben:
- P²,P³: Reste, ausgewählt aus der Gruppe Wasserstoff, C₁-C₃₀-Alkyl, C₁-C₃₀-Acyl, C₃-C₈-Cycloalkyl ggf. substituiert durch ein bis drei C₁-C₆-Alkyl und wobei die Kohlenstoffkette der Alkyl-, Acyl- und Cycloalkylreste durch Sauerstoff in Etherfunktion, Schwefel in Thioetherfunktion oder durch nichtbenachbarte Imino- oder C₁-C₄-Alkyliminogruppen unterbrochen sein kann,
- Y⁹, Y¹⁰: chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y⁹ und Y¹⁰ -O-CO-O-, -O-CO-NR-, -NR-CO-O oder -NR-CO-NR- bedeutet,
- M: eine mesogene Gruppe.

Beispielsweise wären dies Verbindungen wie 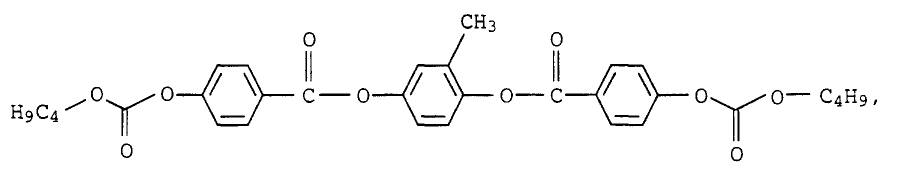 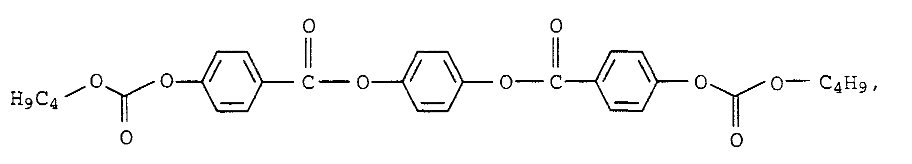 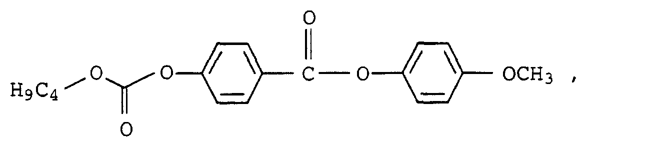 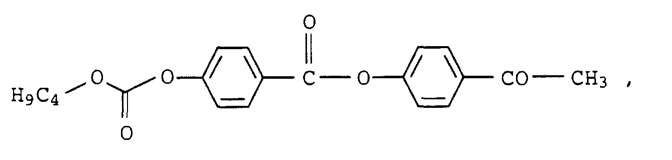 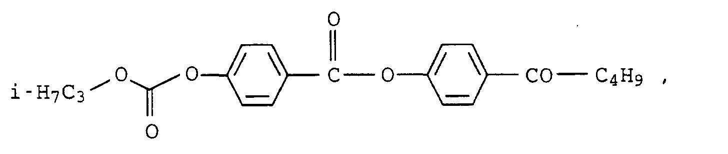 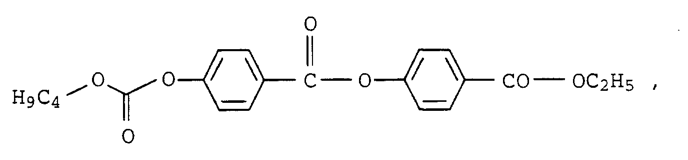 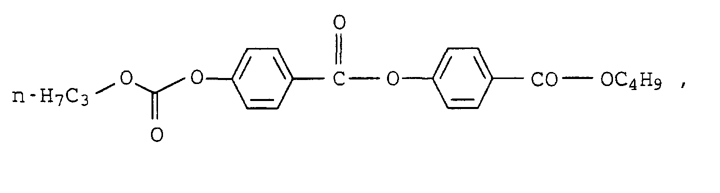 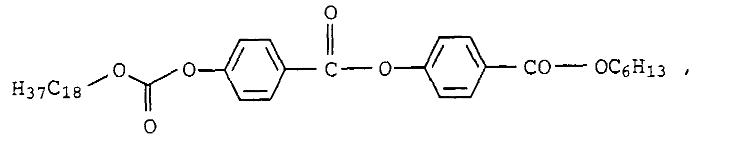 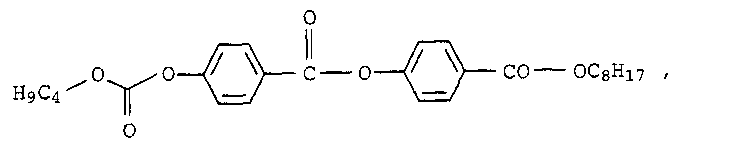 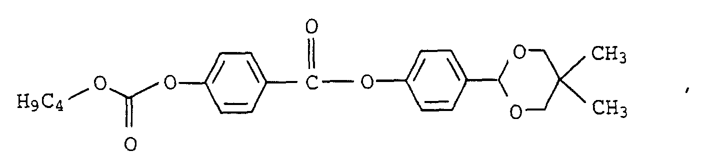 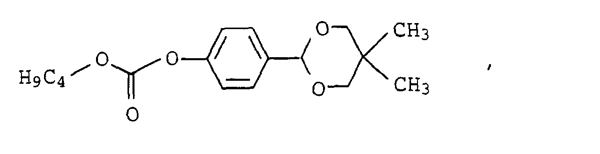 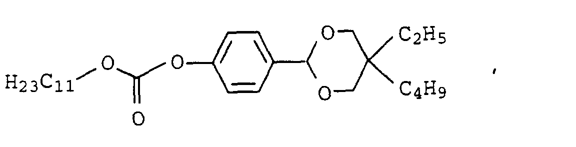 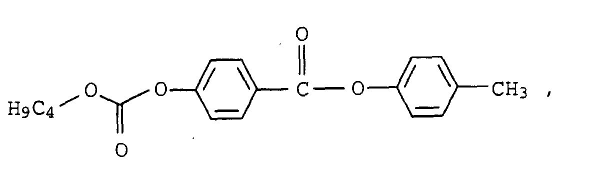 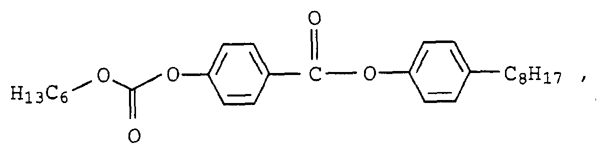 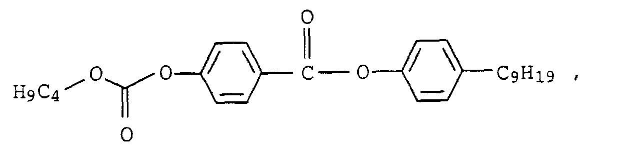 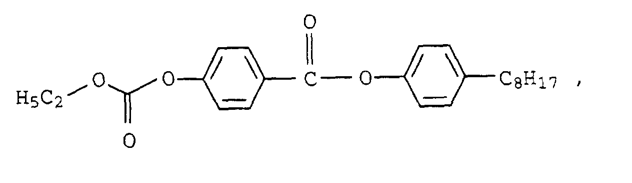 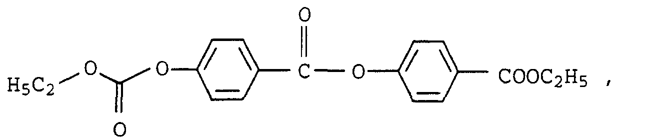 aber auch weitere, zum Teil kommerziell erhältliche, Verbindungen der Formel III.

Werden die erfindungsgemäßen Flüssigkristallzusammensetzungen, welche neben Verbindungen der Formel I als Komponenten auch Verbindungen der Formeln II und III als Komponenten b) bzw. c) enthalten können, polymerisiert, so wird durch den Zusatz letzterer beider Verbindungen die Vernetzungsdichte des entstandenen Polymerisats reduziert. Dadurch lassen sich Eigenschaften wie Härte, Elastizität, Glastemperatur, Flüssigkeits- und Gasdurchlässigkeit usw. der erhaltenen Polymerisationsprodukte einstellen.

Flüssigkristallzusammensetzungen, welche eine oder mehrere der Verbindungen I, II und III enthalten, können zusätzlich auch eine oder mehrere chirale Verbindungen enthalten. Auf diese Weise entstehen cholesterische flüssigkristalline Phasen, die vor allem interessante optische Eigenschaften haben und beispielsweise in Abhängigkeit vom Betrachtungswinkel Licht von unterschiedlicher Wellenlänge reflektieren. Solche Flüssigkristallzusammensetzungen finden besonders als cholesterisch flüssigkristalline Farbmittel Verwendung.

Als chirale Komponenten sind besonders solche geeignet, die einerseits eine große Verdrillungsfähigkeit aufweisen und andererseits gut mischbar mit den flüssigkristallinen Verbindungen sind, ohne die flüssigkristalline Phasenstruktur zu stören.

Bevorzugte chirale Verbindungen sind z.B. solche der allgemeinen Formeln Ib, Ic, Id, Ie

(Z¹-Y⁵)ₙX Ib, (Z¹-Y¹-A¹-Y⁵)ₙX Ic,

(P¹-Y⁵)ₙX Id (Z¹-Y¹-A¹-Y³-M-Y⁴)ₙX Ie,

in der die Variablen die Bedeutung wie für die Formeln I, Ia, II angegeben haben, n für eine Zahl von 1 bis 6 steht und X ein n-wertiger chiraler Rest ist.

Reste X sind beispielsweise 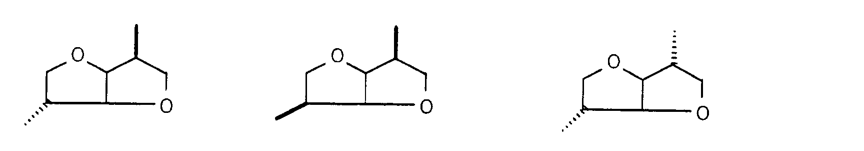 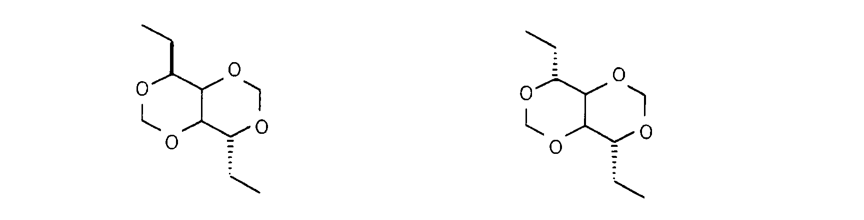 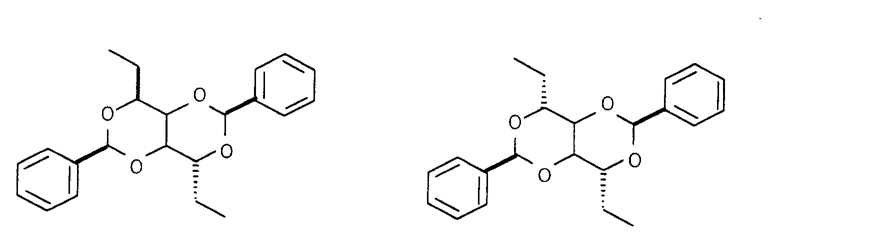 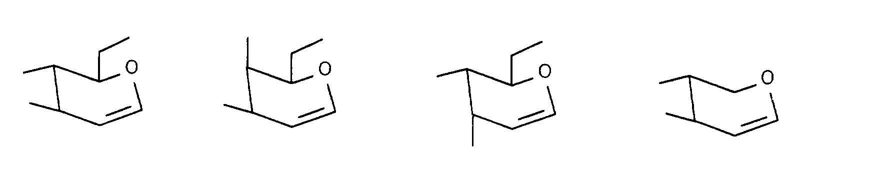 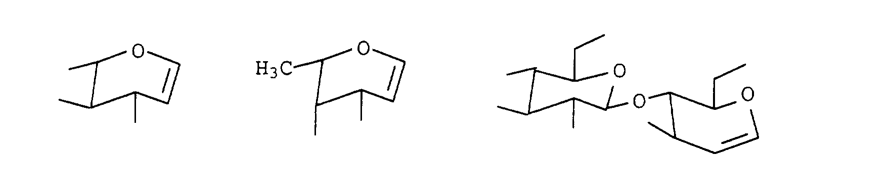 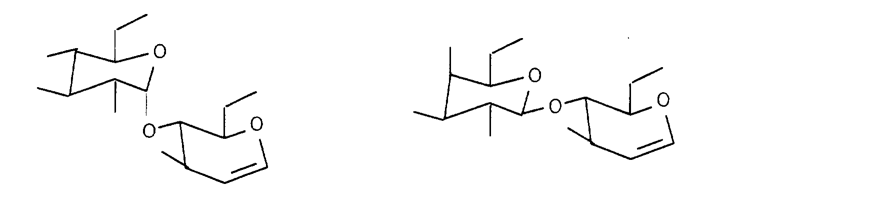 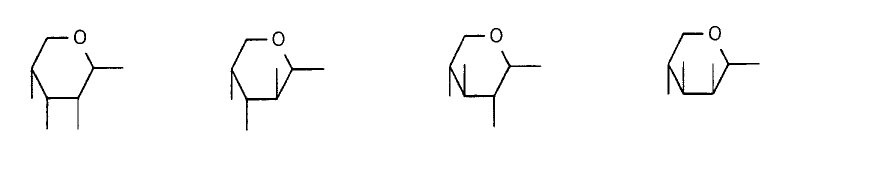 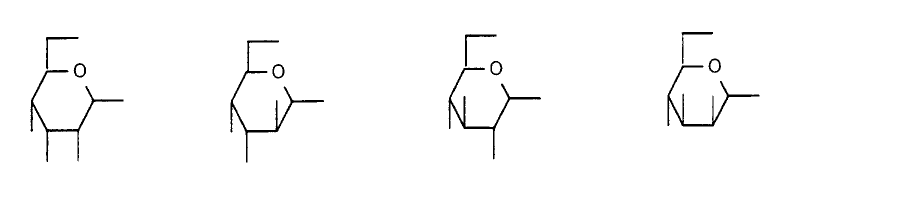 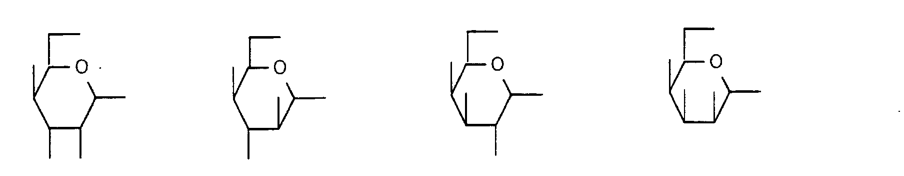 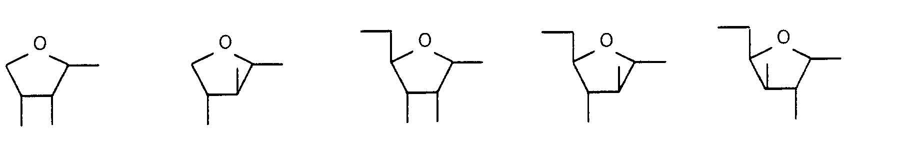 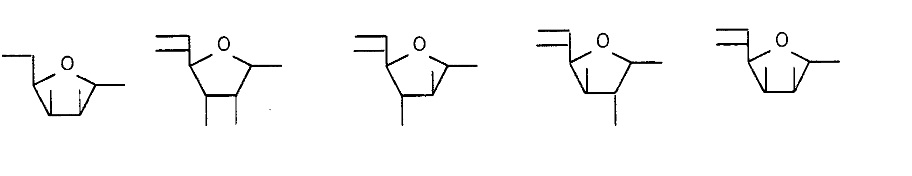 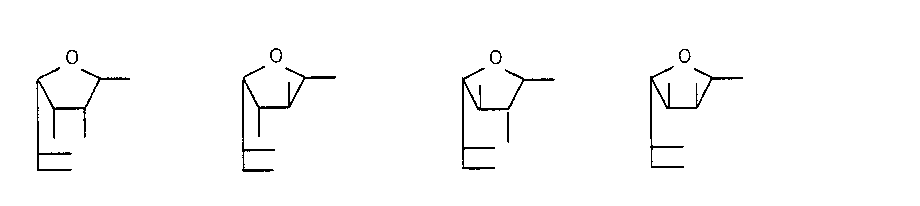 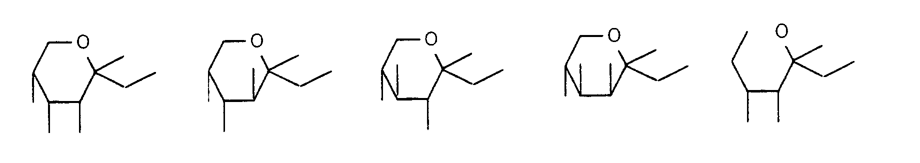 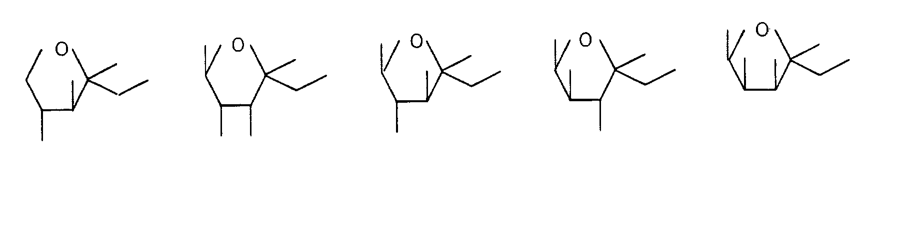 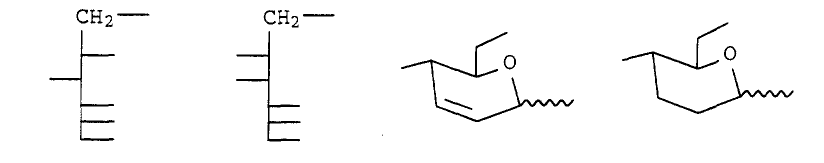 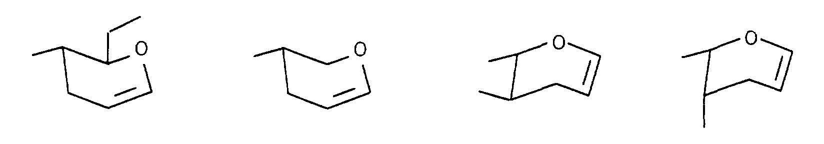 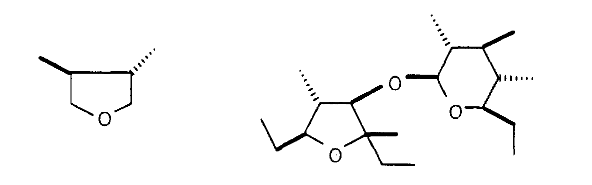 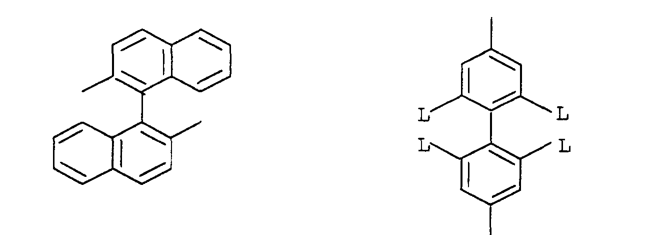 wobei
- L: C₁- bis C₄-Alkyl, C₁-C₄-Alkoxy, Halogen, COOR, OCOR, CONHR oder NHCOR ist und R C₁-C₄-Alkyl bedeutet.
(Die endständigen Striche in den aufgeführten Formeln geben die freien Valenzen an).

Besonders bevorzugt sind z.B.

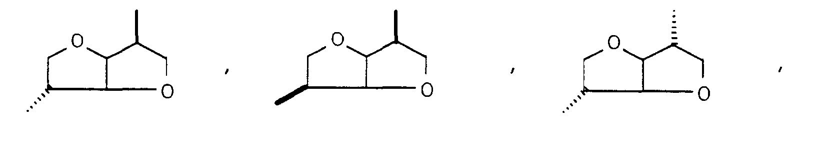

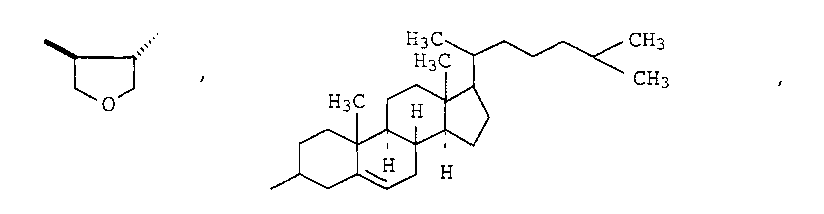

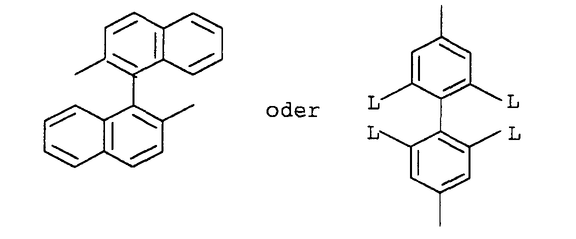

Diese und weitere bevorzugte chirale Komponenten sind beispielsweise in der DE-A 43 42 280 und in den älteren deutschen Patentanmeldungen 19520660.6 und 19520704.1 genannt.

Vorzugsweise werden in den Flüssigkristallzusammensetzungen die Komponenten a), b), c) in einem molaren Anteil an der Gesamtmenge der besagten Komponenten von
a) 1 bis 98 mol-%,
b) 1 bis 98 mol-%,
c) 0,01 bis 90 mol-%,
eingesetzt, wobei die Summe der einzelnen molaren Anteile natürlich 100 mol-% ergeben muß.

Verwendet man Flüssigkristallzusammensetzungen, welche neben den Komponenten a), b), c) auch noch eine oder mehrere chirale Verbindungen enthalten, so werden bevorzugt erstere Komponenten in einem Anteil von 60 bis 99,999 Gew.-% und letztere, chirale Verbindungen in einem Anteil von 0,001 bis 40 Gew.-% gemischt, wobei sich auch hier wieder die Gewichtsanteile auf die Summe der Anteile von Komponenten a), b), c) sowie chiralen Verbindungen beziehen. Die Gesamtanteile ergänzen sich selbstverständlich wieder zu 100 Gew.-%.

Weitere erfindungsgemäße Flüssigkristallzusammensetzungen enthalten 10 bis 100 Gew.-%, bevorzugt 50 bis 100 Gew.-%, besonders bevorzugt 60 bis 100 Gew.-% der Verbindungen I, I und II und/oder III, jeweils bezogen auf das Gesamtgewicht der Flüssigkristallzusammensetzung. Zusätzlich können die Mischungen 0 bis 90 Gew.-%, bevorzugt 0 bis 50 Gew.-% weiterer Monomerer wie die weiter unten beschriebenen Vernetzungsmittel sowie 0 bis 50 Gew.-%, bevorzugt 0 bis 10 Gew.-% einer oder mehrerer polymerisierbarer oder nichtpolymerisierbarer chiraler Verbindungen enthalten.

Polymerisiert man die erfindungsgemäßen Verbindungen oder Flüssigkristallzusammensetzungen, so läßt sich der flüssigkristalline Ordnungszustand fixieren. Die Polymerisation kann je nach polymerisierbarer Gruppe z.B. thermisch oder photochemisch erfolgen. Zusammen mit den erfindungsgemäßen Verbindungen oder Flüssigkristallzusammensetzungen lassen sich dabei auch andere Monomere copolymerisieren. Diese Monomeren können andere polymerisierbare flüssigkristalline Verbindungen, chirale Verbindungen, die ebenfalls bevorzugt kovalent einpolymerisiert werden, oder übliche Vernetzungsmittel wie mehrfachvalente Acrylate, Vinylverbindungen oder Epoxide sein. Besonders im Fall von Isocyanaten, Isothiocyanaten oder Epoxiden als polymerisierbare Flüssigkristallverbindungen ist das Vernetzungsmittel bevorzugt ein mehrfachvalenter Alkohol, so daß beispielsweise Urethane gebildet werden können. Das Vernetzungsmittel muß in seiner Menge so an die Polymerisationsverhältnisse angepaßt werden, daß einerseits eine zufriedenstellende mechanische Stabilität erreicht wird, andererseits aber das flüssigkristalline Phasenverhalten nicht beeinträchtigt wird. Die Menge des Vernetzungsmittels hängt daher von der Verwendung der Polymerisate ab. Zur Herstellung von Pigmenten ist eine größere Menge Vernetzungsmittel vorteilhaft, zur Herstellung thermoplastischer Schichten oder z.B. für Display-Orientierungsschichten ist eine geringere Menge Vernetzungsmittel erforderlich. Die Menge des Vernetzungsmittels kann durch einige Vorversuche ermittelt werden.

Eine weitere Modifizierung der aus den erfindungsgemäßen Verbindungen oder Flüssigkristallzusammensetzungen hergestellten Polymerisationsprodukte ist durch die Zugabe polymerer Hilfsstoffe vor der Polymerisation möglich. Solche Hilfsstoffe sollten vorzugsweise entweder in den Ausgangsmischungen löslich sein oder aber in einem mit den Ausgangsmischungen verträglichen organischen Lösungsmittel. Typische Vertreter solcher polymerer Hilfsstoffe sind z.B. Polyester, Celluloseester, Polyurethane sowie polyether- oder polyestermodifizierte oder auch unmodifizierte Silikone. Die für den gewünschten Zweck gegebenenfalls zuzugebende Menge an polymerem Hilfsstoff, seine chemische Natur sowie möglicherweise noch Menge und Art eines Lösungsmittels sind dem Fachmann in der Regel geläufig oder lassen sich ebenfalls experimentell durch wenige Vorversuche ermitteln.

Neben den Verbindungen der Formeln II (Komponente b) und III (Komponente c) können den polymerisierbaren flüssigkristallinen Verbindungen der Formel I (Komponente a) noch weitere Verbindungen zugemischt werden, welche nichtkovalent in das polymere Netzwerk eingebaut werden. Dies können beispielsweise kommerziell erhältliche nematische Flüssigkristalle sein.

Weitere Zusatzstoffe können auch Pigmente, Farbstoffe sowie Füllstoffe sein.

Bei den Pigmenten können dies sein anorganische Verbindungen wie beispielsweise Eisenoxide, Titanoxid und Ruß, bei den organischen Verbindungen z.B. Pigmente oder Farbstoffe aus den Klassen der Monoazopigmente, Monoazofarbstoffe sowie deren Metallsalze, Disazopigmente, kondensierte Disazopigmente, Isoindolinderivate, Derivate der Naphthalin- oder Perylentetracarbonsäure, Anthrachinonpigmente, Thioindigoderivate, Azomethinderivate, Chinacridone, Dioxazine, Pyrazolochinazolone, Phthalocyaninpigmente oder basische Farbstoffe wie Triarylmethanfarbstoffe und deren Salze.

Als weitere Pigmente kommen in Frage Effektgeber wie Aluminium- oder Glimmerflakes oder auch Pigmente wie z.B. die unter den Namen Iriodin® und Paliocrom® kommerziell erhältlichen Perlglanz- und Effektpigmente.

Weiter können übliche Füllstoffe wie Kreide, Talkum, Gips, Baryt usw. zugesetzt werden.

Die Herstellung der erfindungsgemäßen Verbindungen erfolgt nach an sich bekannten Methoden. Im allgemeinen werden die Molekülteile Z¹, Z², A¹, A² und M durch Kondensationsreaktionen so miteinander verknüpft, daß dabei die Brückenglieder Y¹ bis Y⁴ ausgebildet werden. Die Ausgangskomponenten werden dazu so ausgewählt, daß die entsprechenden Ester oder Amide entstehen. Dieses Reaktionsprinzip gilt auch für den Aufbau der mesogenen Gruppe aus den entsprechenden Ringsystemkomponenten. Die Carbonatgruppe wird bevorzugt durch sukzessive Umsetzung Hydroxylgruppen tragender Molekülteile mit Phosgen gebildet. Carbamatgruppen und Harnstoffgruppen werden entsprechend aus Phosgen und Aminoverbindungen gebildet.

Ein bevorzugter Syntheseweg von Verbindungen der Formel I, in welcher die Variablen Y³ und Y⁴ zugleich -O-CO-O bedeuten, beginnt mit der Umsetzung eines Z¹-CO-Cl Säurechlorides mit einem Spacerdiol unter Ausbildung einer Estergruppe als Y¹:

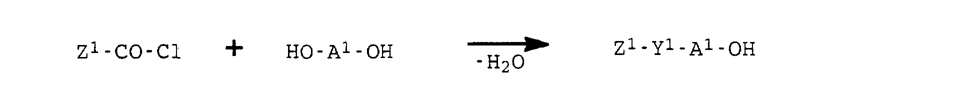

Die resultierende Hydroxyverbindung kann dann mit einer Verbindung Cl-OC-M-CO-Cl oder bevorzugt erst mit einem Äquivalent Phosgen gemäß folgender Reaktion 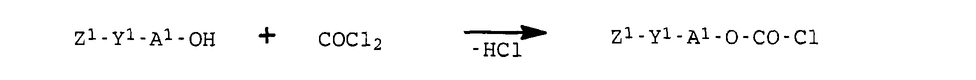 unter Ausbildung des entsprechenden Chlorformiats und anschließend mit einem Mesogendiol zur Zielverbindung umgesetzt werden:

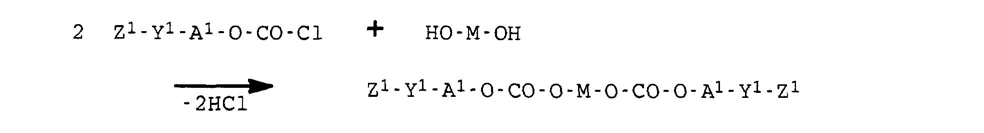

In diesem Fall entsteht eine symmetrische Verbindung in der Z¹-Y¹-A¹-Y³- gleich -Y⁴-A²-Y²-Z² ist. Weiter ist es auch möglich, in einer solchen "Einschrittreaktion" mehrere verschiedene Chlorformiate als Mischung oder an unterschiedlichen räumlichen Stellen nacheinander oder gleichzeitig mit dem Mesogendiol oder einer Mischung von Mesogendiolen umzusetzen. Dabei werden jedoch Mischungen der symmetrischen und unsymmetrischen flüssigkristallinen Verbindungen erhalten. Unsymmetrische Verbindungen lassen sich gezielt durch schrittweise Reaktion mit einem Äquivalent des Mesogendiols und anschließende Umsetzung mit einer Verbindung Cl-CO-O-A²-Y²-Z² erhalten.

Vorteilhaft wird dabei so verfahren, daß abweichend vom stöchiometrischen Molverhältnis von 1:2 für Mesogendiol: (Z¹-Y¹-A¹-O-CO-Cl + Z²-Y²-A²-O-CO-Cl) das Diol in einem molaren Überschuß von 5:2 bis 20:2, vorzugsweise 10:2, in einem Lösungsmittel vorgelegt wird. Das Chlorformiat Z¹-Y¹-A¹-O-CO-Cl wird zugegeben und das überschüssige, nicht umgesetzte Mesogendiol mit einem Fällmittel ausgefällt. Nach dem Abfiltrieren des Feststoffes wird dem Filtrat, welches das sowohl im Lösungs- als auch Fällmittel gut lösliche Zwischenprodukt Z¹-Y¹-A¹-O-CO-O-M-OH enthält, in einem zweiten Schritt das Chlorformiat Z²-Y²-A²-O-CO-Cl zugegeben. Nach üblicher Aufarbeitung erhält man die reine gemischte Verbindung Z¹-Y¹-A¹-Y³-M-Y⁴-A²-Y²-Z², bzw. im Falle einer unsymmetrischen mesogenen Gruppe M oder bei Einsatz mehrerer Mesogendiole ein entsprechendes Isomerengemisch oder eine Mischung von unsymmetrischen Verbindungen I.

Als Lösungsmittel lassen sich dabei in der Regel z.B. Dimethylacetamid, N-Methylpyrrolidon, Dimethylformamid, Methylethylketon oder Aceton, als Fällmittel Methanol, Essigsäureethylester, Dichlormethan oder Butylacetat einsetzen, wobei die Wirkung als Lösung- oder Fällmittel natürlich von den Lösungseigenschaften des Mesogendiols abhängt und zudem das Zwischenprodukt in der Mischung aus Lösungs- und Fällmittel gut löslich sein muß. Für das Beispiel des Mesogendiols 1,4-(4,4'Bishydroxybenzoyloxy)-2-methylbenzol 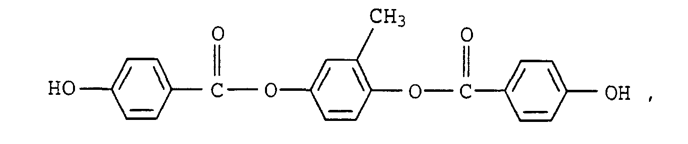 ist die Kombination von Dimethylformamid und Essigsäureethylester als Lösungs- bzw. Fällmittel gut geeignet.

Weiter wird zum Abfangen des entstehenden HCl vorteilhaft vor jedem Chlorformiat-Zugabeschritt eine Hilfsbase mindestens äquimolar zu den Chlorformiat-Mengen zugegeben. Dabei kommen in Frage tertiäre Amine wie z.B. Trimethyl-, Triethyl- oder auch N,N-Dimethylcyclohexylamin, Pyridin oder auch anorganische Basen wie Alkali- oder Erdalkalicarbonate oder -hydrogencarbonate sowie Mischungen aus organischen und anorganischen Basen.

Als weitere Basen können auch Alkali- oder Erdalkaliacetate eingesetzt werden. Bevorzugt werden N,N-Dimethylcyclohexylamin und Kaliumcarbonat sowohl alleine als auch in Mischung zugegeben.

Die Umsetzungstemperaturen liegen im allgemeinen zwischen 0°C und 60°C, meist im Bereich von 40°C bis 50°C. Je nach Reaktivität der Umsetzungspartner ergeben sich Reaktionsdauern zwischen 3 und 24 Stunden.

Zur Aufarbeitung wird üblicherweise der Reaktionsansatz mit Wasser und einem mit Wasser wenig oder nicht mischbaren organischen Extraktionsmittel verdünnt, die organische Phase mehrmals mit Wasser und schließlich mit wäßriger Mineralsäure nachgewaschen.

Die Entfernung des organischen Extraktionsmittels erfolgt mittels Vakuumdestillation bei Temperaturen von 20°C bis 40°C. Zur Verhinderung der vorzeitigen Polymerisation des Produkts/der Produkte werden vor der destillativen Aufarbeitung übliche Inhibitoren wie Methoxyphenol, Kerobit® BHT oder Phenothiazin, meist in Mischung, in Mengen von 0,01 bis 1 Gew.-%, bezogen auf das Produkt/die Produkte, zugegeben. Die Mischung der unterschiedlich flüchtigen Inhibitoren gewährleistet dabei eine ausreichende Stabilisierung des Produkts/der Produkte sowohl in der Flüssig- als auch Dampfphase.

Als organische Extraktionsmittel kommen vorzugsweise Toluol oder Essigsäureethylester zum Einsatz. Zur sauren Nachwäsche wird bevorzugt konzentrierte Salzsäure verwendet.

Für die Umsetzung der Mesogendiole mit den Chlorformiaten lassen sich bevorzugt zusätzlich noch Verdünnungsmittel zusetzen. Diese bewirken aufgrund der Herabsetzung der Viskosität der Reaktionsmischung eine schnellere Vermischung der Reaktionspartner und damit eine Verkürzung der Umsetzungsdauer. Als vorteilhafte Verdünnungsmittel lassen sich beispielsweise Toluol, Xylol, Ethylacetat, Butylacetat oder auch Tetrahydrofuran oder die verschiedensten isomeren Dioxane einsetzen. Weiterhin kann das Verdünnungsmittel selbst auch als Base wirken, wie beispielsweise die oben erwähnten tertiären Amine oder auch Pyridin.

Die erfindungsgemäße Herstellung der Verbindungen der Formel II, in welchen die Variablen Y⁷ und Y⁸ -O-CO-O- bedeuten, verläuft analog zur gezielten, schrittweisen Herstellung der gemischten Verbindungen Z¹-Y¹-A¹-O-CO-O-M-O-CO-O-A²-Y²-Z². Dabei kann das Mesogendiol entweder im ersten Schritt mit dem Chlorformiat Z³-Y⁶-A³-O-CO-Cl und im zweiten Schritt mit P¹-O-CO-Cl umgesetzt werden oder die Zugabe der Chlorformiate erfolgt in umgekehrter Reihenfolge.

Soll eine Flüssigkristallzusammensetzung hergestellt werden, welche neben Verbindung I auch noch die Verbindungen II und III enthält, so läßt sich diese primär durch Mischen der Einzelverbindungen in den bereits oben angeführten bevorzugten Anteilen herstellen.

Ein einfacher Weg besteht in der Umsetzung eines oder mehrerer Diole mit einem oder mehreren Chlorformiaten der Formel Z¹-Y¹-A¹-O-CO-Cl sowie mit einem oder mehreren Chlorformiaten der Formel P¹-O-CO-Cl. Dabei werden die Chlorformiate entweder an räumlich getrennten Stellen gleichzeitig oder bereits als Mischung zugegeben. Sollen bestimmte Verbindungen I, II, III gezielt im Über- oder Unterschuß hergestellt werden, so ist auch die schrittweise Zugabe der verschiedenen Chlorformiate zweckmäßig.

Bevorzugt wird zur Herstellung solcher Flüssigkristallzusammensetzungen ein Molverhältnis von Chlorformiat(en) Z¹-Y¹-A¹-O-CO-Cl zu Chlorformiat(en) P¹-O-CO-Cl von 99:1 bis 20:80, vorzugsweise von 99:1 bis 40:60. Besonders bevorzugt wird ein Verhältnis von 50:50 gewählt.

Werden Mischungen der Chlorformiate eingesetzt und sind die Reaktivitäten der Chlorformiate sowohl untereinander wie auch gegenüber dem Diol HO-M-OH bzw. der entsprechenden Monohydroxyzwischenstufe (einfach substituiertes Mesogendiol) gleich, so läßt sich die (statistische) Verteilung der Verbindungen I, II, III entsprechend den nachstehenden Ausführungen ermitteln. Zur Vereinfachung sei an dieser Stelle definiert:

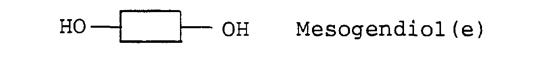

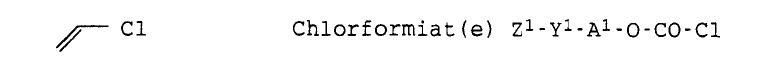

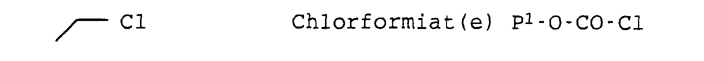

Durch Umsetzung des Diols (der Diole) mit einer Mischung von Chlorformiaten im Molverhältnis 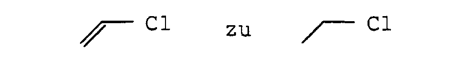 von 99:1 erhält man im Idealfall nach der Erstsubstitution folgende Verteilung

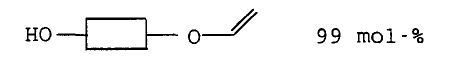

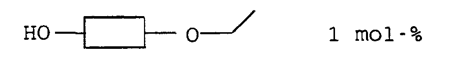

Jede dieser Zwischenverbindungen wird wiederum im obigen Verhältnis von 99:1 zweitsubstituiert, d.h.

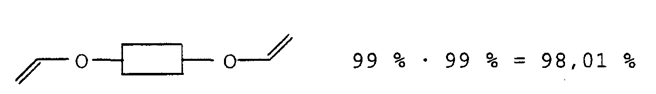

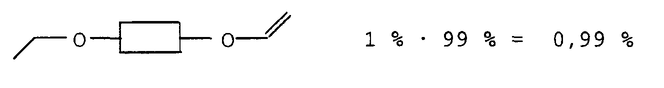

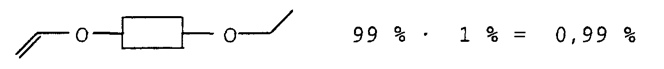

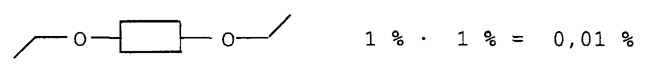

Sind die beiden gemischt substituierten Produkte gleich, d.h. ist das Mesogendiol symmetrisch, so ergibt sich ein Verhältnis von Verbindungen I zu II zu III von 98,01 %:1,98 % (= 2·0,99 %):0,01 %.

Analog erhält man für eine entsprechende Chlorformiatmischung im Molverhältnis 20:80 bzw. 40:60 bzw. 50:50 ein Molverhältnis der Verbindungen I, II und III zueinander von 4 %:32 % (= 2.16 %):64 % bzw. 16 : 48 % (= 2·24 %):36 % bzw. 25 %:50 % (= 2·25 %):25 %.

In ähnlicher Weise können die verschiedenen anderen Gruppen Y¹ - Y⁴ ausgebildet werden, wobei bevorzugt Säurehalogenide mit den entsprechenden Amino- oder Hydroxyverbindungen umgesetzt werden. Sauerstoff- und Schwefelbrücken werden in bekannter Weise nach den Methoden der Ethersynthese in die Verbindungen eingeführt.

Weitere Details zur Herstellung der Verbindungen sind in den Schriften WO 95/22586, WO 95/24454 und WO 95/24455 angegeben.

Die erfindungsgemäßen Flüssigkristallzusammensetzungen eignen sich in hervorragender Weise zur Beschichtung von Oberflächen. Ein Verfahren zur Erzeugung solcher Beschichtungen mit flüssigkristallinem Ordnungszustand ist dadurch gekennzeichnet, daß man die erfindungsgemäßen Fflüssigkristallzusammensetzungen, welche gegebenenfalls weitere polymerisierbare Verbindungen und chirale Verbindungen enthalten, gewünschtenfalls mit einem Verdünnungsmittel zur Herabsetzung der Viskosität verdünnt, auf ein Substrat aufbringt, eine flüssigkristalline Orientierung herbeiführt und dann die auf das Substrat aufgebrachten Verbindungen polymerisiert.

Die flüssigkristalline Orientierung entsteht entweder spontan während des Auftrages oder sie wird durch bekannte physikalische Methoden wie z.B. Rakeln oder Anlegen eines elektrischen oder magnetischen Feldes erreicht.

Wird beispielsweise an den Einsatz der erfindungsgemäßen Flüssigkristallzusammensetzung im Bereich des Siebdrucks gedacht, so läßt sich deren Viskosität nicht nur durch den erwähnten Zusatz von Verdünnungsmitteln oder durch die Verwendung von Mischungen erfindungsgemäßer Verbindungen I und II, sondern auch durch die Anteile an nichtvernetzender Verbindung III reduzieren. Höhere Viskositäten sind im Gegensatz dazu beispielsweise im Bereich der Automobillackierungen tolerierbar oder sogar erwünscht.

Die erfindungsgemäßen Verbindungen oder Flüssigkristallzusammensetzungen finden beispielsweise auch bei der Herstellung optischer Anzeigegeräte insbesondere zur Herstellung von Orientierungsschichten in Flüssigkristall-Displays Verwendung. Auch als polymerisierbare Matrixkomponente für polymer dispergierte Displays können die Verbindungen oder Zusammensetzungen verwendet werden.

Weitere Verwendungsmöglichkeiten sind photovernetzbare Klebstoffe auf Flüssigkristallbasis.

Besonders bevorzugt ist die Verwendung der erfindungsgemäßen Verbindungen oder Flüssigkristallzusammensetzungen in cholesterischen Flüssigkristallmischungen, die als flüssigkristalline Farbmittel dienen. Besonders zu nennen sind in diesen Zusammensetzungen Fahrzeuglacke auf der Basis cholesterischer Flüssigkristallsysteme. Diese Lacke können entweder durch direkte Beschichtung der Substrate hergestellt werden oder sie können Pigmente enthalten, welche die erfindungsgemäßen Verbindungen in einem durch Polymerisation fixierten cholesterisch flüssigkristallinen Ordnungszustand enthalten.

Weiter lassen sich die erfindungsgemäßen Flüssigkristallzusammensetzungen auch zur Herstellung von Pigmenten verwenden. Dabei wird die Flüssigkristallzusammensetzung polymerisiert und das entstandene Polymerisat mittels üblicher Aggregate zerkleinert und klassiert. Vorzugsweise wird dabei die Ausgangszusammensetzung als dünne Schicht ausgerakelt und gewünschtenfalls zusätzlich im elektrischen oder magnetischen Feld ausgerichtet. Auf diese Weise sind auch plättchenförmige Pigmente zugänglich.

### Beispiele

| Zeichenerklärung: | |
|---|---|
| n.b.nicht bestimmt | cr kristallin |
| s smektisch | i isotrop |
| n nematisch | ch cholesterisch |

Zur Charakterisierung des Phasenverhaltens wird folgende Notation verwendet:
- Beispiel a):: cr 78-83 n 87-88 i bedeutet,
daß die kristalline Phase(cr) zwischen 78 und 83°C mit der nematischen Phase (n) koexistent ist. Zwischen 87 und 88°C ist das Koexistenzgebiet zwischen nematischer und isotroper Phase(i). Oberhalb 88°C liegt eine isotrope, "geklärte" Schmelze vor.
- Beispiel b) :: cr 77 i bedeutet, daß die kristalline Phase (cr) bei 77°C in eine isotrope Phase(i) ≙ Schmelze übergeht.

Das Phasenverhalten der Verbindungen bzw. Mischungen wurde polarisationsmikroskopisch untersucht. Die Temperatur-Kontrolle erfolgte mittels eines Heiztisches vom Typ FP 80/82 der Fa. Mettler.

Viskositäten wurden mit einem "Rheometrics Dynamic Spectrometer" (Fa. Rheometrics) in Kegel-Platte-Geometrie bestimmt.

An einigen Mischungen wurden mittels Flachrakel Aufstriche angefertigt, welche entweder visuell oder mittels eines Spektrometers (Hitachi U-2000, Fa. Hitachi) beurteilt wurden. Bei der visuellen Beurteilung bedeutet dabei beispielsweise "rot-grün", daß der Aufstrich in senkrechter Aufsicht eine rote, in streifender Aufsicht eine grüne Farbe zeigte. Die gemessenen Wellenlängen λ(┴) beziehen sich auf das reflektierte Licht in senkrechter Aufsicht.

### Präparationen:

### Herstellvorschrift 1

Herstellung der Verbindungen (Beispiele 1 bis 14)

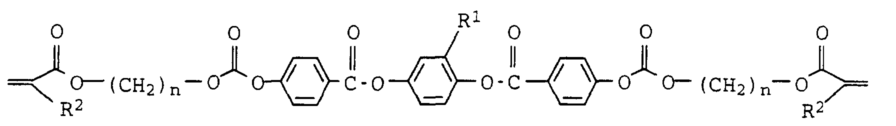

Eine Lösung aus 5 mmol 1,4-Bis[4'-hydroxybenzoyloxy]-2-R¹-benzol (Mesogendiol) und 20 ml Pyridin wurde bei 0°C tropfenweise mit einer Lösung aus 12 mmol eines Chlorformiats 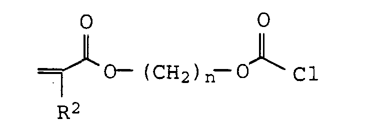 in 5 ml Dichlormethan versetzt, wonach das Reaktionsgemisch 3 Stunden lang bei Raumtemperatur gerührt wurde. Anschließend wurde verdünnte Salzsäure zugegeben, wobei das Produkt als Feststoff ausfiel. Dieser wurde abfiltriert, gewaschen und durch Umkristallisation gereinigt.

Die Einzelheiten dieser Versuche sowie deren Ergebnisse sind der nachfolgenden Tabelle 1 zu entnehmen.

**Tabelle 1**

| Bsp. | R¹ | R² | n | Flüssigkristalliner Temperaturbereich [°C] |
|---|---|---|---|---|
| 1 | H | H | 2 | 124 - 162 |
| 2 | H | H | 4 | 71 ->100 (polymerisiert) |
| 3 | H | H | 6 | n.b. |
| 4 | H | H | 8 | 69 - 136 |
| 5 | CH₃ | H | 2 | 117 - 166 |
| 6 | CH₃ | H | 4 | 58 - 119 |
| 7 | CH₃ | H | 6 | 53 - 110 |
| 8 | CH₃ | H | 8 | 46 - 80 |
| 9 | Cl | H | 2 | 80 - 120 |
| 10 | Cl | H | 4 | <20 - 95 |
| 11 | Cl | H | 6 | <20 - 51 |
| 12 | Cl | H | 8 | 45 - 100 |
| 13 | H | CH₃ | 2 | 155 - 169 |
| 14 | H | CH₃ | 4 | 103 ->130 (polymerisiert) |

### Herstellvorschrift 2

### Aa) Herstellung der Verbindung (Beispiel 15)

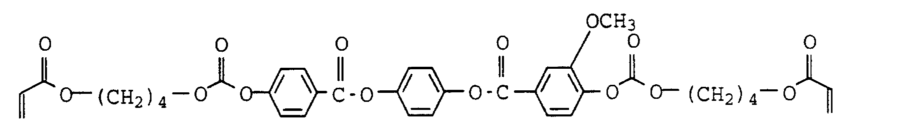

95,1 g (0,25 mol) 1-(3'-Methoxy-4'-hydroxybenzoyloxy)-4-(4'-hydroxybenzoyloxy)benzol wurden in 182 ml Dimethylformamid und 95,25 g (0,75 mol) Dimethylcyclohexylamin gelöst und bei Raumtemperatur 124,96 g (0,605 mol) Acryloxybutyloxychlorformiat innerhalb von 30 Minuten hinzugetropft. Dann wurde 3,5 h bei 40°C und über Nacht bei Raumtemperatur nachgerührt. Nach Zugabe von Wasser wurde mit Essigsäureethylester verdünnt, mit konz. Salzsäure auf einen pH-Wert von 1 gestellt, die wäßrige Phase abgetrennt und die organische Phase zweimal mit Wasser gewaschen. Nach Trocknen der organischen Phase wurde der Essigsäureethylester im Vakuum entfernt und das Rohprodukt durch Filtration über Kieselgel mit dem Eluent Petrolether/Essigsäureethylester 2:1 gereinigt. Das Produkt zeigte folgendes Phasenverhalten:
cr 78-83 n 87-88 i

### Ab) Herstellung der Verbindung (Beispiel 16)

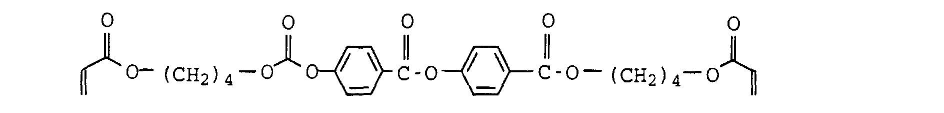

96,1 g (0,25 mol) der Verbindung 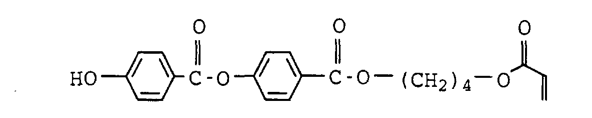 wurden in 182 ml Dimethylformamid und 47,6 g (0,375 mol) Dimethylcyclohexylamin gelöst und bei Raumtemperatur 62,5 g (0,303 mol) Acryloxybutyloxychlorformiat innerhalb von 30 Minuten zugetropft. Dann wurde 3,5 h bei 40°C und über Nacht bei Raumtemperatur nachgerührt. Nach Zugabe von Wasser wurde mit Essigsäureethylester verdünnt, mit konz. Salzsäure auf einen pH-Wert von 1 gestellt, die wäßrige Phase abgetrennt und die organische Phase zweimal mit Wasser gewaschen. Nach Trocknen der organischen Phase wurde der Essigsäureethylester im Vakuum entfernt und das Rohprodukt durch Filtration über Kieselgel mit dem Eluent Petrol-ether/Essigsäurethylester 2:1 gereinigt. Man erhielt ein öliges Reinprodukt.

### Ac) Herstellung der Verbindungen (Beispiele 17 bis 22)

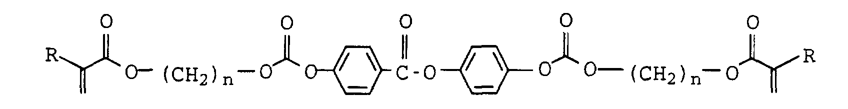

57,5 g (0,25 mol) 4-(4'-Hydroxybenzoyloxy-)phenol wurden in 182 ml Dimethylformamid und 95,25 g (0,75 mol) Dimethylcyclohexylamin gelöst und bei Raumtemperatur 0,605 mol des Chlorformiats 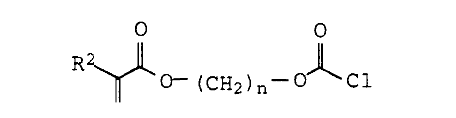 innerhalb von 30 Minuten hinzugetropft. Dann wurde 3,5 h bei 40°C und über Nacht bei Raumtemperatur nachgerührt. Nach Zugabe von Wasser wurde mit Essigsäureethylester verdünnt, mit konz. Salzsäure auf einen pH-Wert von 1 gestellt, die wäßrige Phase abgetrennt und die organische Phase zweimal mit Wasser gewaschen. Nach Trocknen der organischen Phase wurde der Essigsäureethylester im Vakuum entfernt und das Rohprodukt durch Filtration über Kieselgel mit dem Eluent Petrolether/Essigsäureethylester 2:1 gereinigt.

Die Einzelheiten zu den Versuchen sind der nachfolgenden Tabelle 2 zu entnehmen.

**Tabelle 2**

| Bsp. | R | n | Phasenverhalten |
|---|---|---|---|
| 17 | H | 2 | n.b. |
| 18 | H | 4 | cr 41 i |
| 19 | H | 6 | n.b. |
| 20 | CH₃ | 2 | n.b. |
| 21 | CH3 | 4 | n.b. |
| 22 | CH₃ | 6 | n.b. |

### Ba) Herstellung der Verbindungen (Beispiele 23 bis 27)

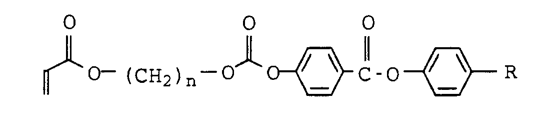

0,25 mol der Verbindung 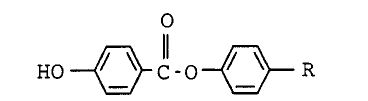 wurden in 182 ml Dimethylformamid und 47,6 g (0,375 mol) Dimethylcyclohexylamin gelöst und bei Raumtemperatur 0,303 mol des Chlorformiats 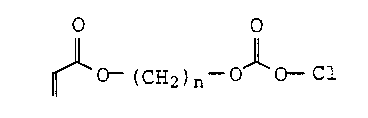 innerhalb von 30 Minuten hinzugetropft. Dann wurde 3,5 h bei 40°C und über Nacht bei Raumtemperatur nachgerührt. Nach Zugabe von Wasser wurde mit Essigsäureethylester verdünnt, mit konz. Salzsäure auf einen pH-Wert von 1 gestellt, die wäßrige Phase abgetrennt und die organische Phase zweimal mit Wasser gewaschen. Nach Trocknen der organischen Phase wurde der Essigsäureethylester im Vakuum entfernt und das Rohprodukt durch Filtration über Kieselgel mit dem Eluent Petrolether/Essigsäureethylester 2:1 gereinigt.

Die Einzelheiten zu den Versuchen sind der nachfolgenden Tabelle 3 zu entnehmen.

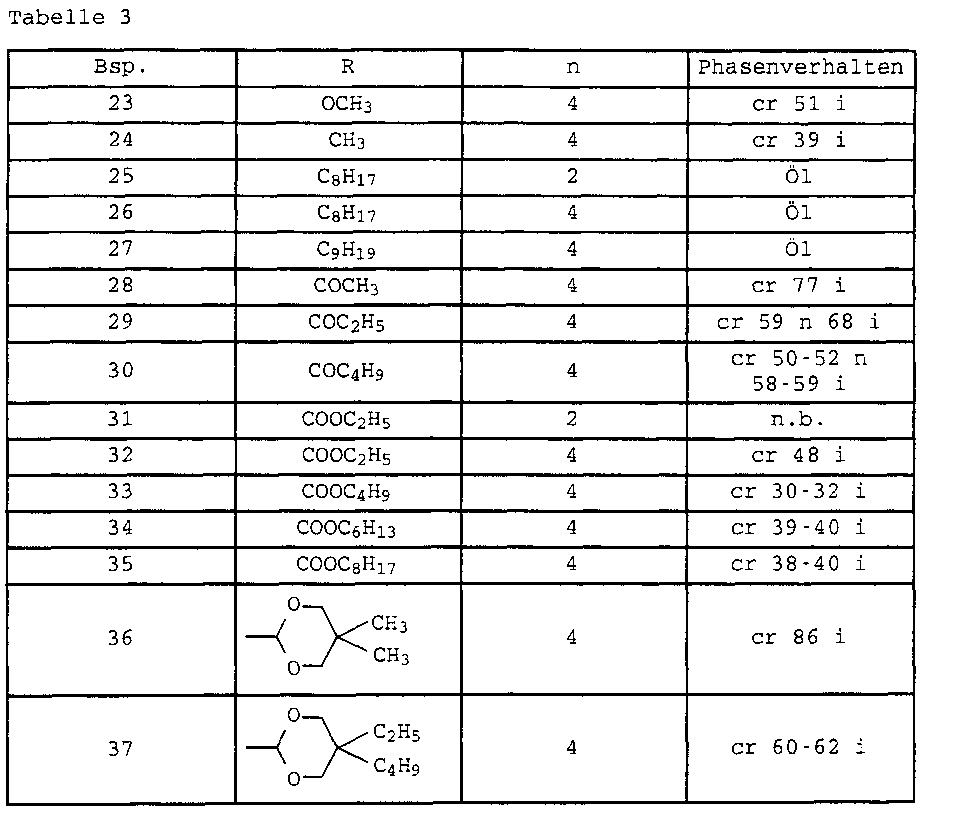

### Bb) Herstellung der Verbindungen (Beispiele 38 bis 40)

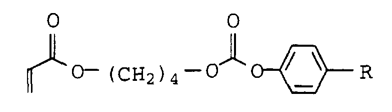

0,25 mol der Verbindung 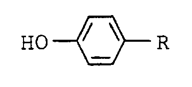 wurden in 182 ml Dimethylformamid und 47,6 g (0,375 mol) Dimethylcyclohexylamin gelöst und bei Raumtemperatur 62,5 g (0,303 mol) Acryloxybutyloxychlorformiat innerhalb von 30 Minuten zugetropft. Dann wurde 3,5 h bei 40°C und über Nacht bei Raumtemperatur nachgerührt. Nach Zugabe von Wasser wurde mit Essigsäureethylester verdünnt, mit konz. Salzsäure auf einen pH-Wert von 1 gestellt, die wäßrige Phase abgetrennt und die organische Phase zweimal mit Wasser gewaschen. Nach Trocknen der organischen Phase wurde der Essigsäureethylester im Vakuum entfernt und das Rohprodukt durch Filtration über Kieselgel mit dem Eluent Petrolether/Essigsäureethylester 2:1 gereinigt.

Die Einzelheiten zu den Versuchen sind in der nachstehenden Tabelle 4 aufgeführt.

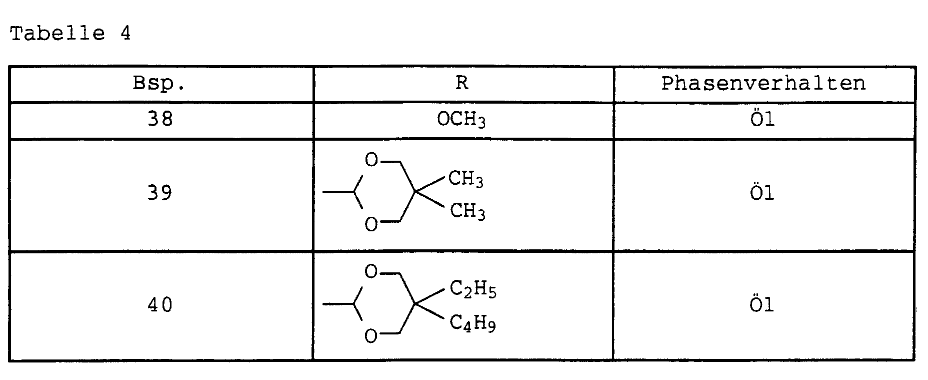

### Herstellvorschrift 3

### Herstellung der Verbindung (Beispiel 6)

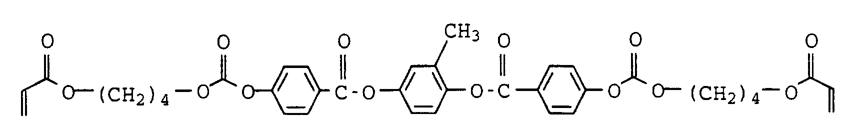

364 g (1,0 mol) 1,4-Bis[4'-hydroxybenzoyl]-2-methylbenzol und 303,5 g (2,2 mol) Kaliumcarbonat wurden in 1 Liter N-Methylpyrrolidon suspendiert und bei Raumtemperatur 495,6 g (2,4 mol) Acryloxybutyloxychlorformiat innerhalb von 30 Minuten hinzugegeben. Es wurde 30 Minuten bei 40°C nachgerührt, dann 151,0 g (1 mol) Dimethylcyclohexylamin hinzugefügt und weitere 3 h bei 40°C nachgerührt. Anschließend wurden 2 Liter Toluol beigemengt und zweimal mit je 2 Liter Wasser ausgerührt. Nach Ablassen der wäßrigen Phase wurden 2 Liter Wasser hinzugegeben und mit 128 g konz. Salzsäure auf einen pH-Wert von 1 eingestellt. Anschließend wurde wiederum zweimal mit je 2 Liter Wasser ausgerührt. Nach Abtrennen der wäßrigen Phase wurden 85 mg Kerobit BHT und 85 mg Methoxyphenol zugegeben und das Toluol im Vakuum bei max. 40°C Badtemperatur abgedampft. Die Rohausbeute betrug 730,6 g. Nach Filtration über Kieselgel mit Petrolether/Essigsäureethylester als Eluent wurden 678,7 g (96 %) 1,4-Bis[4'acryloxybutylbenzoyl]2-methylbenzol erhalten (Phasenverhalten: cr 58 n 122 i).

### Herstellvorschrift 4

A) Selektive Herstellung der unsymmetrischen Verbindungen (Beispiele 41 und 42) 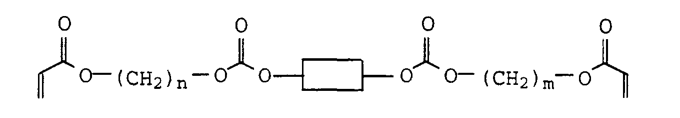 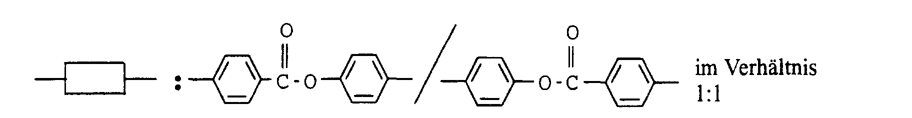 23 g (0,1 mol) 4-(4-Hydroxybenzoyloxy-)phenol wurden in 100 g Dimethylformamid gelöst und 1,2 g (0,01 mol) Dimethylcyclohexylamin hinzugefügt. Dann wurden 0,01 mol des Chlorformiats 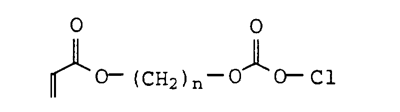 bei Raumtemperatur hinzugefügt. Es wurde 4 h bei 40°C nachgerührt und der Reaktionsansatz mit 200 ml Essigsäureethylester versetzt. Der ausgefallene Feststoff (überschüssiges 4-(4-Hydroxybenzoyloxy-)phenol) wurde abgesaugt und das Filtrat auf 80°C erhitzt. Nach Abdestillieren von ca. 180 ml Essigsäureethylester wurden zu der Mutterlauge 0,01 mol des Chlorformiats 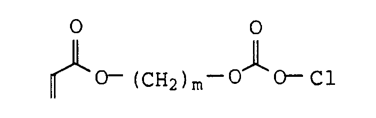 und weitere 1,2 g (0,01 mol) Dimethylcyclohexylamin hinzugegeben und 4 h bei 40°C nachgerührt. Die Reaktionsmischung wurde auf 200 ml Wasser gegossen und dreimal mit je 100 ml Essigsäureethylester extrahiert. Die vereinigten organischen Phasen wurden über Natriumsulfat getrocknet und nach Entfernen des Lösungsmittels das Rohprodukt chromatographisch gereinigt. Man erhielt eine Mischung der beiden isomeren Diacrylate 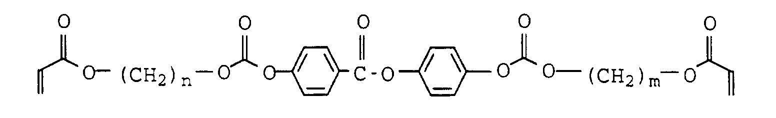 und 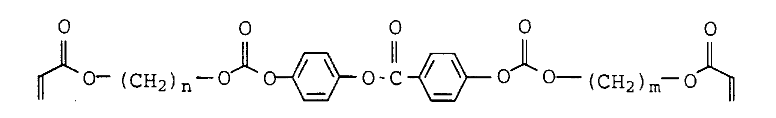 Tabelle 5 enthält die Einzelheiten zu den Versuchen.

**Tabelle 5**

| Bsp. | n | m | Phasenverhalten |
|---|---|---|---|
| 41 | 2 | 4 | n.b. |
| 42 | 4 | 6 | n.b. |

B) Selektive Herstellung der unsymmetrischen Verbindung (Beispiel 43) 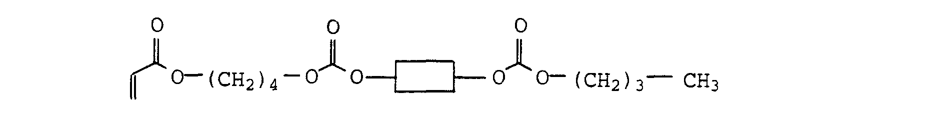 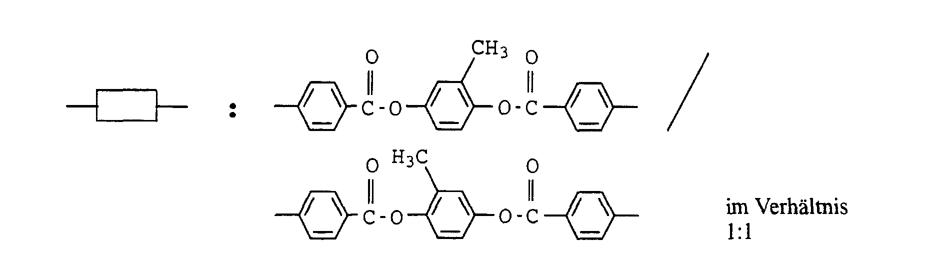 36,4 g (0,1 mol) 1,4-Bis[4'-hydroxybenzoyloxy]-2-methylbenzol wurden in 100 g Dimethylformamid gelöst und 1,2 g (0,01 mol) Dimethylcyclohexylamin hinzugefügt. Dann wurden 2,08 g (0,01 mol) Acryloxybutyloxychlorformiat bei 15 bis 20°C hinzugefügt. Es wurde 4 h bei 40°C nachgerührt und der Reaktionsansatz mit 200 ml Essigsäureethylester versetzt. Der ausgefallene Feststoff wurde abgesaugt und das Filtrat auf 80°C erhitzt. Nach Abdestillation von ca. 180 ml Essigsäureethylester wurden zu der Mutterlauge 1,36 g (0,01 mol) Butyloxychlorformiat und weitere 1,2 g (0,01 mol) Dimethylcyclohexylamin hinzugegeben und 4 h bei 40°C nachgerührt. Die Reaktionsmischung wurde auf 200 ml Wasser gegossen und dreimal mit je 100 ml Essigsäureethylester extrahiert. Die vereinigten organischen Phasen wurden über Natriumsulfat getrocknet und nach Entfernen des Lösungsmittels das Rohprodukt chromatographisch gereinigt. Man erhielt 5,72 g (90 %) der beiden isomeren Monoacrylate 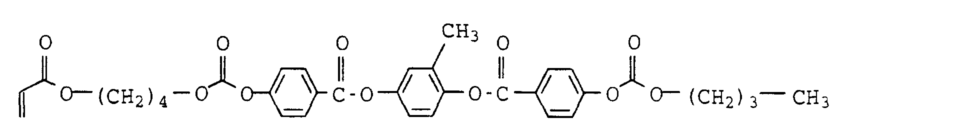 und 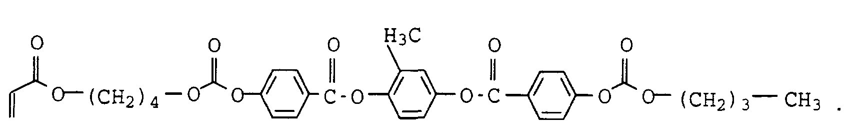 Phasenverhalten: n 134 i

### Herstellvorschrift 5

Aa) Herstellung von statistischen Mischungen von Verbindungen (Beispiele 44 bis 46) 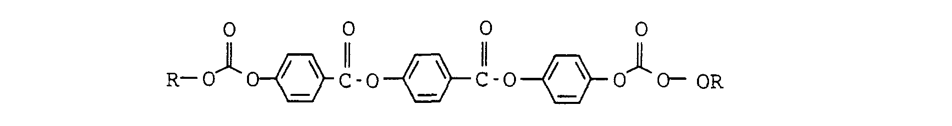 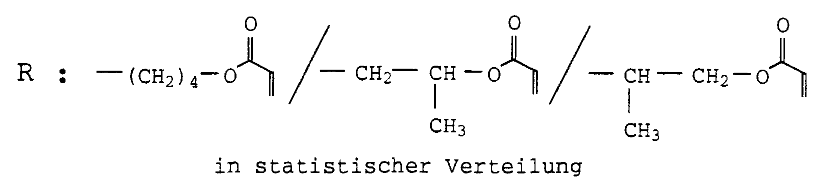 350 g (1,0 mol) 1,4-Bis[4'-hydroxybenzoyloxy-]benzol wurden in 728 ml Dimethylformamid eingetragen und 381 g (3 mol) Dimethylcyclohexylamin hinzugefügt. Zwischen 0°C und 5°C wurde eine Mischung von 2,1 mol Acryloxybutyloxychlorformiat (Chlorformiat CF1) und 2-Acryloxy-2-methylethyloxychlorformiat/2-Acryloxy-1-methylethyloxychlorformiat (Chlorformiat CF2; die beiden isomeren Verbindungen fallen bei der Herstellung des Chlorformiats in Mischung an, die relativen Anteile der 2- bzw. 1-Methylverbindung ließen sich nicht bestimmen) im Molverhältnis der Butyl- zur Mischung der isomeren Methylethylverbindungen von 70:30 bzw. 80:20 bzw. 90:10 innerhalb von 30 Minuten zugetropft. Die Reaktionsmischung wurde 3 h bei 40°C nachgerührt.
   Der Reaktionsansatz wurde mit 2 Liter Toluol und 1 Liter Wasser verdünnt und mit 120 ml konzentrierter Salzsäure sauer gestellt. Nach Ablassen der wäßrigen Phase wurde zweimal mit je 500 ml Wasser neutral gewaschen. Nach Zugabe von 63,5 mg Kerobit® BHT, 63,5 mg Methoxyphenol und 126,8 mg Phenothiazin zur organischen Phase erfolgte die Abtrennung des Toluols durch Vakuumdestillation bei einer Badtemperatur von 40°C.
   Die Versuchsergebnisse sind in nachfolgender Tabelle 6 zusammen-gefaßt. 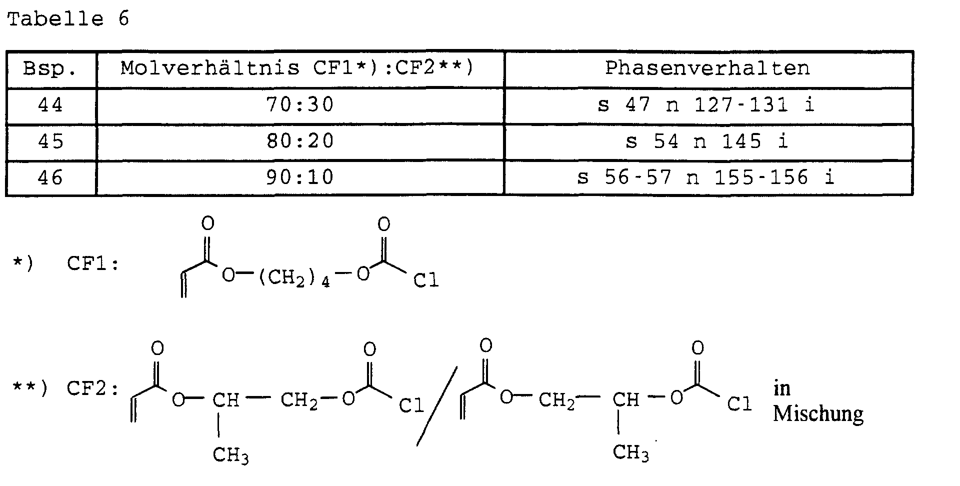
Ab) Analog zu Aa) wurde eine statistische Mischung von Verbindungen (Beispiel 47) 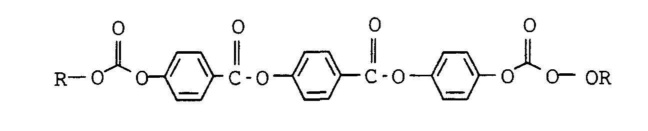 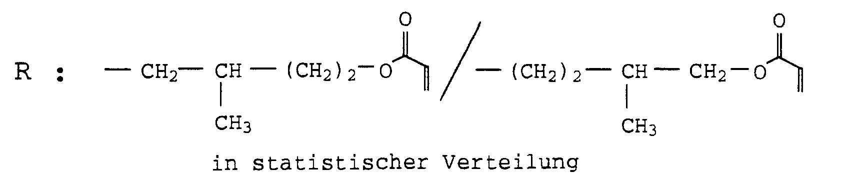 durch Umsetzung von 1,0 mol 1,4-Bis[4'-hydroxbenzoyloxy-]benzol mit 2,1 mol einer 1:1-Mischung der Chlorformiate 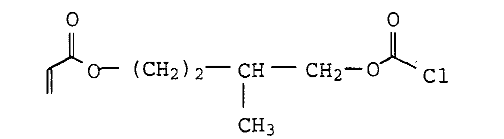 und 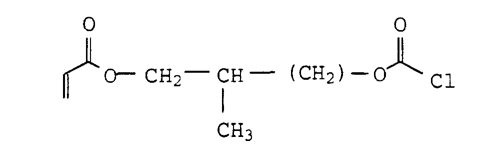 erhalten. Die Mischung der Chlorformiate läßt sich herstellen durch Umsetzung des Diols 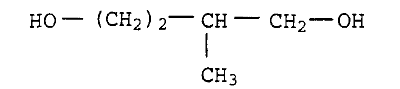 mit je einem Äquivalent Acrylsäurechlorid und Phosgen.
   Eine Analyse der resultierenden Mischung der o.g. Verbindungen erfolgte nicht, doch wird man Anteile der beiden symmetrischen Verbindungen 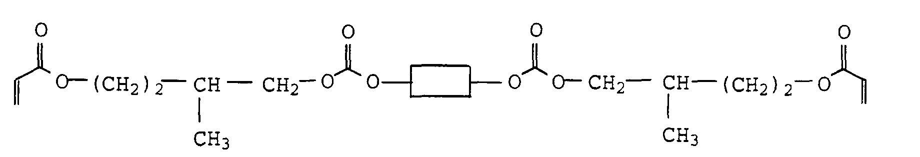 und 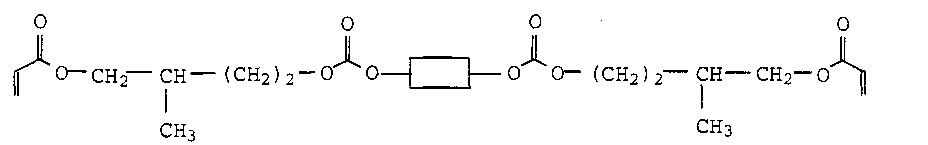 sowie der unsymmetrischen Verbindung 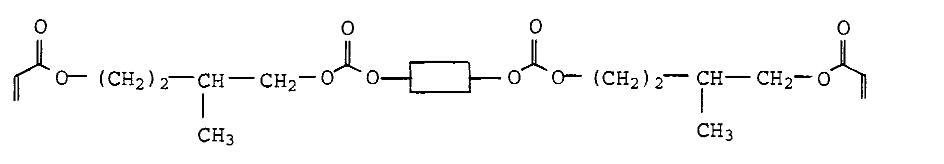 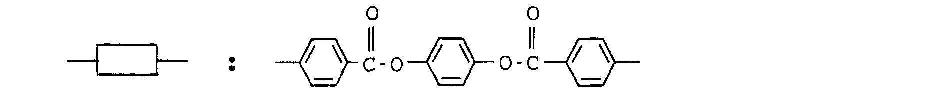 im Molverhältnis von (nahezu) 25:25:50 erwarten.
B) Herstellung von statistischen Mischungen von Verbindungen (Beispiele 48 und 49)

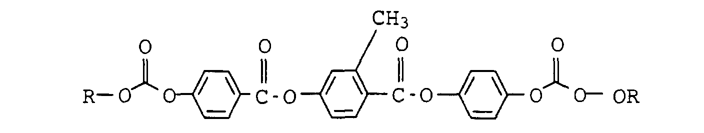

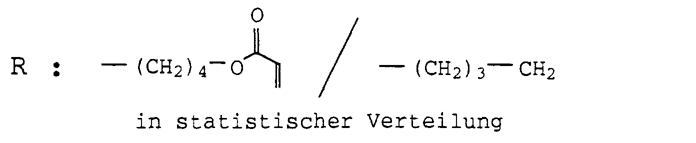

364 g (1,0 mol) 1,4-Bis[4'-hydroxybenzoyloxy-]benzol wurde in 728 ml Dimethylformamid eingetragen und 381 g (3 mol) Dimethylcyclohexylamin hinzugefügt. Zwischen 0°C und 5°C wurde eine Mischung von 2,1 mol Acryloxybutyloxychlorformiat (Chlorformiat CF1) und Butyloxy-chlorformiat (Chlorformiat CF3) im Molverhältnis von 50:50 bzw. 90:10 innerhalb von 30 Minuten zugetropft. Die Reaktionslösung wurde 3 h bei 40°C nachgerührt.

Der Reaktionsansatz wurde mit 2 Liter Toluol und 1 Liter Wasser verdünnt und mit 120 ml konzentrierter Salzsäure sauer gestellt. Nach Ablassen der wäßrigen Phase wurde zweimal mit je 500 ml Wasser neutral gewaschen. Nach Zugabe von 63,5 mg Kerobit® BHT, 63,5 mg Methoxyphenol und 126,8 mg Phenothiazin zur organischen Phase erfolgte die Abtrennung des Toluols durch Vakuumdestillation bei einer Badtemperatur von 40°C.

Die Versuchsergebnisse sind in nachfolgender Tabelle 7 zusammen-gefaßt.

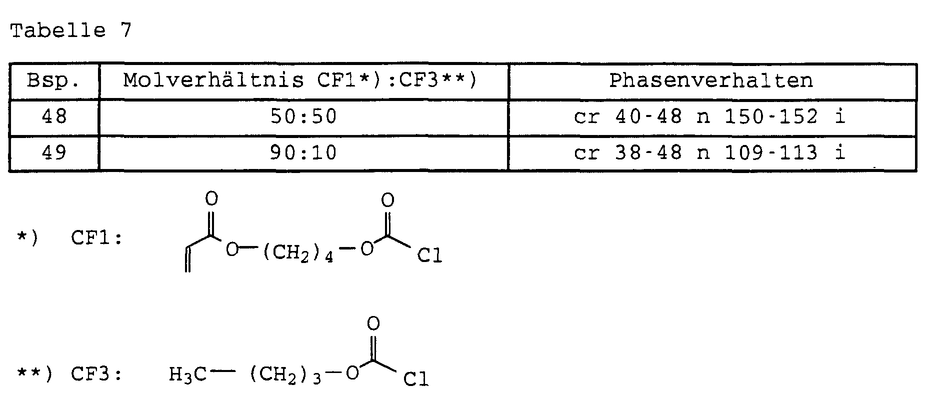

Die Mischungen der Beispiele 48 und 49 wurden chromatografisch analysiert. Dabei ergaben sich die in Tabelle 8 aufgeführten Molprozente der einzelnen Mischungskomponenten
a) (entspricht einer erfindungsgemäßen verbindung der Formel I) 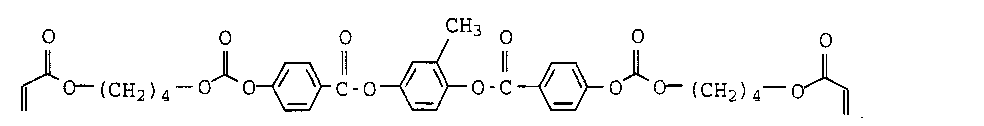
b) (entspricht einer erfindungsgemäßen Verbindung der Formel II) 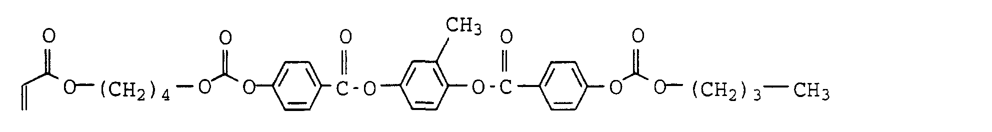 bzw. 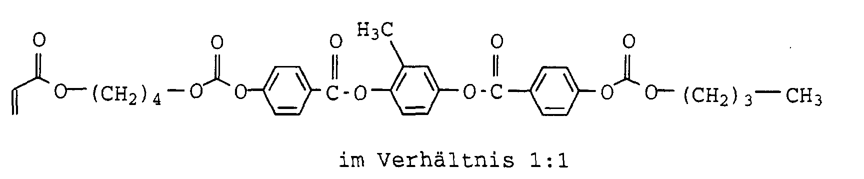
c) (entspricht einer Verbindung der Formel III) 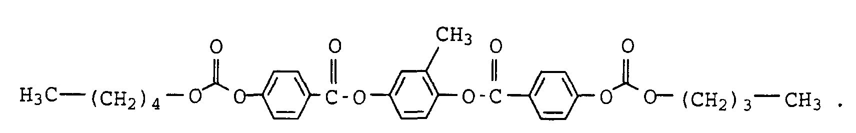 Als Vergleich sind zusätzlich die für die Komponenten a, b und c berechneten Molprozente in Tabelle 8 aufgelistet, wie sie sich aus den Molverhältnissen der eingesetzten Chlorformiate CF1 und CF3 ableiten lassen.

**Tabelle 8**

| Komponente (Mol-%) | | Beispiel 48 | Beispiel 49 |
|---|---|---|---|
| a | bestimmt | 28 | 78 |
| | berechnet | 25 | 81 |
| b | bestimmt | 48 | 20 |
| | berechnet | 50(2·25) | 18(2·9) |
| c | bestimmt | 24 | 2 |
| | berechnet | 25 | 2 |

### Mischungen:

Die Zahlenwerte in den nachstehenden Tabellen verstehen sich als Gew.-% der aufgeführten Mischungskomponenten.

Eine Spaltenüberschrift "Bsp. 6" bzw. "Bsp. 49" bedeutet beispielsweise, daß ein in der entsprechenden Spalte genauer spezifizierter Anteil des Produkts aus dem Herstellbeispiel 6 bzw. einer statistischen Mischung aus dem Herstellbeispiel 49 eingesetzt wurde.

Bei den mit "Luc", "Irga" und "Daro" abgekürzten Mischungszusätzen handelt es sich um die kommerziell erhältlichen Photoinitiatoren Lucirin® TPO, Irgacure® 184 und Darocure® 1173. Die chemische Identität der unter den anderen Abkürzungen aufgeführten Zusätze ist wie folgt.

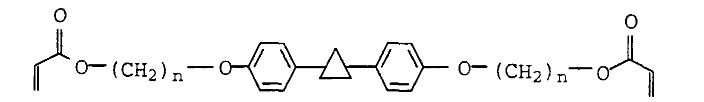

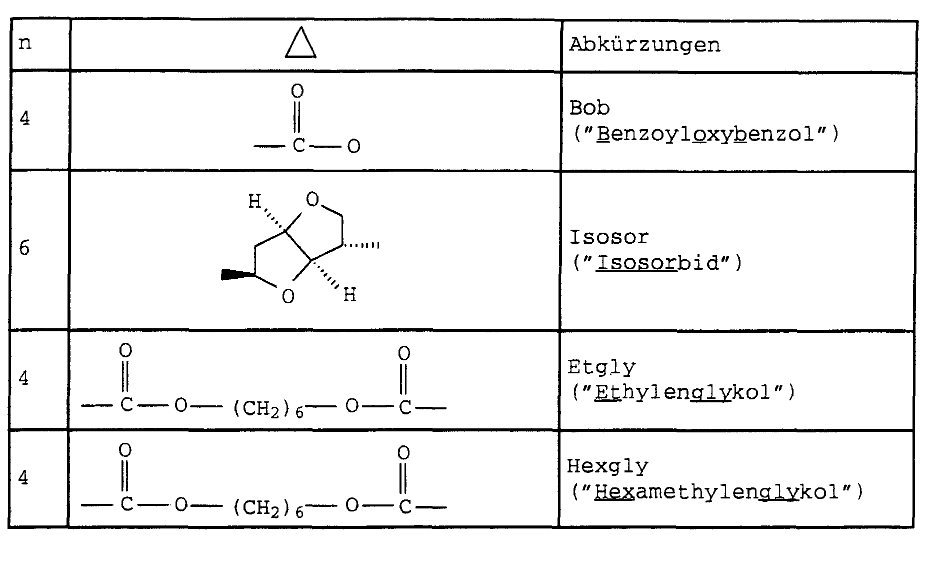

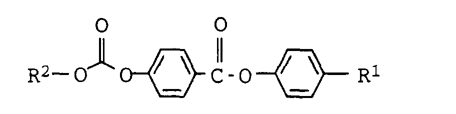

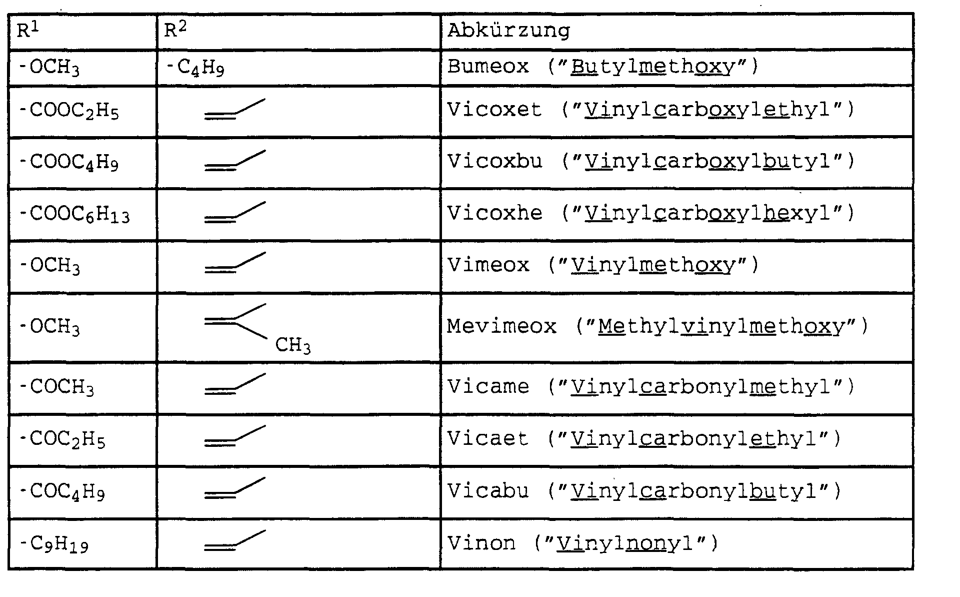

**Tabelle 9**

| Mischung | Bsp.2 | Bsp.6 | Bsp.18 | Bob | Phasenverhalten |
|---|---|---|---|---|---|
| 1 | 85.1 | | | 14.9 | cr 31-64 n 130-138 i |
| 2 | 68.2 | | | 31.8 | cr 32-58 n 110-114 i |
| 3 | 48.8 | | | 51.2 | cr 32-42 n 89-95 i |
| 4 | 38.0 | | | 62.0 | cr 31-46 n 67-71 i |
| 5 | 26.4 | | | 73.6 | cr 32-48 n 70-74 i |
| 6 | | 85.4 | | 14.6 | cr 40-67 n 107-111 i |
| 7 | | 68.7 | | 31.3 | cr 40-54 n 91-96 i |
| 8 | | 49.3 | | 50.7 | cr 35-46 n 72-77 i |
| 9 | 82.9 | | 17.1 | | s 56-64 n 140-142 i |
| 10 | 64.5 | | 35.5 | | s 46-58 n 116-119 i |
| 11 | 54.8 | | 45.2 | | s 46-51 n 98-104 i |
| 12 | 44.7 | | 55.3 | | s 46-49 n 76-80 i |

**Tabelle 10**

| Mischung | Bsp.45 | Bsp.23 | Bsp.30 | Isosor | Bumeox | Luc | Phasenverhalten |
|---|---|---|---|---|---|---|---|
| 13 | 89.2 | 10.8 | | | | | s 39-40 n 113-119 i |
| 14 | 79.4 | 20.6 | | | | | n 103-105 i |
| 15 | 52.4 | 47.6 | | | | | n 52-55 i |
| 16 | 41.5 | 58.5 | | | | | n 58-61 i |
| 17 | 29.3 | 70.7 | | | | | n 52-55 i |
| 18 | 28.2 | 39.7 | 32.1 | | | | n 62-63 i |
| 19 | 27.4 | 21.2 | 51.4 | | | | n 68-69 i |
| 20 | 58.2 | | | | 41.8 | | s 49-50 n 93-94 i |
| 21 | 30.4 | 55.1 | | | 14.5 | | n 53-55 i |
| 22 | 81.3 | 9.8 | | 8.9 | | | s 40-41 ch 85-93 i |
| 23 | 74.5 | 19.3 | | 6.2 | | | ch 78 i |
| 24 | 72.9 | 18.9 | | 6.1 | | 2.1 | ch 68 i |
| 25 | 72.0 | 18.6 | | 9.4 | | | ch 69-79 i; λ(┴)=546 nm |
| 26 | 72.0 | 18.7 | | 7.3 | | 2.0 | ch 75 i |
| 27 | 71.5 | 18.5 | | 10.0 | | | ch 75-80 i |
| 28 | 70.2 | 18.2 | | 9.2 | | 2.4 | ch 60 i; λ(┴)=563 nm |
| 29 | 69.8 | 18.1 | | 9.7 | | 2.4 | ch 67-73 i; λ(┴)=527 nm |
| 30 | 38.4 | 54.1 | | 7.5 | | | ch 41-46 i |
| 31 | 37.4 | 52.8 | | 7.3 | | 2.5 | ch 31 i |
| 32 | 94.4 | | | 5.6 | | | s 48 ch 112-118 i |
| 33 | 92.1 | | | 5.4 | | 2.5 | s 56 ch 113-118 i |

**Tabelle 11**

| Mischung | Bsp.48 | Bsp.6 | Bsp.18 | Bsp.23 | Isosor | Irga | Luc | Toluol | Phasenverhalten |
|---|---|---|---|---|---|---|---|---|---|
| 34 | 72.2 | | 27.8 | | | | | | n 116-119 i |
| 35 | 62.6 | | 37.4 | | | | | | n 98-104 i |
| 36 | 52.7 | | 47.3 | | | | | | n 93-98 i |
| 37 | 42.6 | | 57.4 | | | | | | n 76-81 i |
| 38 | 57.9 | | 34.7 | | 7.4 | | | | ch 84-87 i |
| 39 | 55.7 | | 33.3 | | 7.1 | 3.9 | | | rot-grün |
| 40 | 55.0 | | 32.9 | | 8.3 | 3.8 | | | grün-blau |
| 41 | 86.0 | | | 14.0 | | | | | n 117-123 i |
| 42 | 69.7 | | | 30.3 | | | | | n 98-102 i |
| 43 | 50.6 | | | 49.4 | | | | | n 75-79 i |
| 44 | 45.2 | | | 54.8 | | | | | n 70-73 i |
| 45 | 39.7 | | | 60.3 | | | | | n 64 i |
| 46 | 23.5 | | | 76.5 | | | | | n 43-48 i |
| 47 | 45.3 | 25.1 | | 29.6 | | | | | n 100-104 i |
| 48 | 41.4 | 23.0 | | 27.1 | 8.5 | | | | ch 78-87 i |
| 49 | 46.8 | | | 45.8 | 7.4 | | | | ch 55-67 i |
| 50 | 45.7 | | | 44.6 | 7.2 | | 2.5 | | ch 47 i |
| 51 | 45.0 | | | 44. 0 | 7.1 | | 2.4 | 1.5 | ch 48i; λ(┴)=555 nm; Fließviskosität: 3 Pa·s |
| 52 | 43.3 | | | 49.2 | 7.5 | | | | ch 56-64 i; λ(┴)=539 nm |
| 53 | 41.8 | | | 50.6 | 7.6 | | | | ch 53-60 i |
| 54 | 40.8 | | | 49.4 | 7.4 | | 2.4 | | ch 42-51 i; λ(┴)=572 nm |

**Tabelle 12**

| Mischung | Bsp.48 | Bsp.23 | Bsp.24 | Bsp.38 | Isosor | Bob | Luc | Phasenverhalten |
|---|---|---|---|---|---|---|---|---|
| 55 | 87.6 | 5.1 | | 7.3 | | | | n 109-115 i |
| 56 | 86.9 | 7.7 | | 5.4 | | | | n 110-117 i |
| 57 | 62.7 | 28.6 | | 8.7 | | | | n 72-76 i |
| 58 | 54.5 | 26.6 | | 18.9 | | | | n 44-50 i |
| 59 | 52.4 | 38.5 | | 9.1 | | | | n 60-66 i |
| 60 | 58.0 | 26.5 | | 8.1 | 7.4 | | | ch 49-55 i |
| 61 | 44.5 | 43.5 | | 4.4 | 7.6 | | | ch 46-53 i |
| 62 | 43.4 | 42.4 | | 4.3 | 7.5 | | 2.4 | ch 35 i |
| 63 | 82.7 | | 17.3 | | | | | n 114-119 i |
| 64 | 65.6 | | 34.4 | | | | | n 88-93 i |
| 65 | 46.2 | | 53.8 | | | | | n 59-65 i |
| 66 | 34.7 | | 65.3 | | | | | n 42-49 i |
| 67 | 98.0 | | | 2.0 | | | | n 127-132 i |
| 68 | 84.8 | | | 15.2 | | | | n 81-95 i |
| 69 | 78.2 | | | 21.8 | | | | n 65-76 i; Fließviskosität: 3 Pa·s |
| 70 | 70.6 | | | 29.4 | | | | n 41-50 i; Fließviskosität: 1.3 Pa·s |
| 71 | 84.1 | | | | | 15.9 | | cr 31-54 n 120-130 i |
| 72 | 66.4 | | | | | 33.6 | | cr 31-61 n 100-109 i |
| 73 | 46.0 | | | | | 54.0 | | cr 31-69 n 80-84 i |
| 74 | 24.8 | | | | | 75.2 | | cr 50 n 67-72 i |

**Tabelle 13**

| Mischung | Bsp. 49 | Bsp. 23 | Bsp. 38 | Bsp. 43 | Isosor | Daro | Luc | Toluol | Xylol | Phasenverhalten |
|---|---|---|---|---|---|---|---|---|---|---|
| 75 | 88.9 | 10.1 | | | | | | | | n 97-99 i; Fließviskosität: 9 Pa·s |
| 76 | 70.4 | 29.6 | | | | | | | | n 79-83 i; Fließviskosität: 5 Pa·s |
| 77 | 50.0 | 50.0 | | | | | | | | n 60-62 i; Fließviskosität: 3 Pa·s |
| 78 | 24.3 | 75.7 | | | | | | | | n 36-42 i; Fließviskosität: 2 Pa·s |
| 79 | 73.7 | 15.4 | 10.9 | | | | | | | n 59-64 i |
| 80 | 73.2 | 20.2 | 6.6 | | | | | | | n 65-70 i |
| 81 | 72.1 | 22.6 | 5.3 | | | | | | | n 68-72 i |
| 82 | 66.5 | 27.7 | | | 5.8 | | | | | ch 67-72 i; rot-grün |
| 83 | 65.7 | 27.3 | | | 7.0 | | | | | ch 63-69 i; rot-grün |
| 84 | 65.9 | 27.4 | | | 6.7 | | | | | ch 65 i |
| 85 | 64.6 | 26.9 | | | 9.5 | | | | | ch 57 i |
| 86 | 47.9 | 44.8 | | | 7.3 | | | | | ch 46-54 i |
| 87 | 63.0 | 26.2 | | | 6.4 | | 2.4 | | 2.0 | ch 57 i; λ(┴)=704 nm |
| 88 | 61.2 | 25.5 | | | 8.0 | | 2.4 | 2.9 | | ch 50 i; λ(┴)=533 nm; Fließviskosität: 3 Pa·s |
| 89 | 61.3 | 25.5 | | | 8.0 | 3.7 | | 1.5 | | ch 42 i; λ(┴)=533 nm; Fließviskosität: 3 Pa·s |
| 90 | 60.8 | 25.3 | | 3.5 | 8.0 | 2.4 | | | | ch 44 i |

**Tabelle 14**

| Mischung | Bsp.49 | Bsp.24 | Bsp.28 | Bsp.29 | Bsp.31 | Bsp.32 | Bsp.33 | Phasenverhalten |
|---|---|---|---|---|---|---|---|---|
| 91 | 86.7 | 13.3 | | | | | | n 95-99 i |
| 92 | 71.4 | 28.6 | | | | | | n 75-80 i |
| 93 | 52.6 | 47.3 | | | | | | n 54-76 i |
| 94 | 29.4 | 70.6 | | | | | | n 29-32 i |
| 95 | 86.2 | | 13.8 | | | | | n 106-121 i |
| 96 | 69.0 | | 31.0 | | | | | n 92-106 i |
| 97 | 49.7 | | 50.3 | | | | | cr 52 n 77-92 i |
| 98 | 85.2 | | | 14.8 | | | | n 106-119 i |
| 99 | 77.6 | | | 22.4 | | | | n 99-104 i; Fließviskosität: 10.6 Pa·s |
| 100 | 68.3 | | | 31.7 | | | | n 98-112 i |
| 101 | 58.1 | | | 41.9 | | | | n 85-87 i; Fließviskosität: 10.6 Pa·s |
| 102 | 48.9 | | | 51.1 | | | | n 88-101 i |
| 103 | 37.2 | | | 62.8 | | | | n 72-75 i |
| 104 | 84.3 | | | | 15.7 | | | n 102-104 i |
| 105 | 63.6 | | | | 36.4 | | | n 69-72 i |
| 106 | 40.7 | | | | 59.3 | | | n 34-55 i |
| 107 | 78.7 | | | | | 21.3 | | n 98-103 i |
| 108 | 66.6 | | | | | 33.4 | | n 79-83 i |
| 109 | 77.6 | | | | | | 22.4 | n 88-91 i |
| 110 | 59.5 | | | | | | 40.5 | n 59-63 i |

**Tabelle 15**

| Mischung | Bsp. 49 | Bsp. 34 | Bsp. 35 | Bsp. 36 | Bsp. 37 | Bsp. 38 | Bsp. 39 | Bsp. 40 | Phasenverhalten |
|---|---|---|---|---|---|---|---|---|---|
| 111 | 81.2 | 19.8 | | | | | | | n 91-93 i |
| 112 | 65.6 | 34.4 | | | | | | | n 69-73 i |
| 113 | 40.9 | 59.1 | | | | | | | n 36-38 i |
| 114 | 80.8 | | 19.2 | | | | | | n 89-92 i |
| 115 | 58.3 | | 41.2 | | | | | | n 55-61 i |
| 116 | 79.8 | | | 20.2 | | | | | n 103-106 i; Fließviskosität: 15.7 Pa·s |
| 117 | 73.7 | | | 26.3 | | | | | n 87-90 i; Fließviskosität: 11.7 Pa·s |
| 118 | 50.8 | | | 49.2 | | | | | n 67-69 i; Fließviskosität: 10.6 Pa·s |
| 119 | 81.1 | | | | 18.9 | | | | n 97-100 i |
| 120 | 59.0 | | | | 41.0 | | | | n 74-77 i |
| 121 | 40.4 | | | | 59.6 | | | | n 53-57 i |
| 122 | 95.8 | | | | | 4.2 | | | n 96-99 i |
| 123 | 75.2 | | | | | 24.8 | | | n 34-43 i; Fließviskosität: 1.3 Pa·s |
| 124 | 79.1 | | | | | | 20.9 | | n 68-70 i |
| 125 | 76.2 | | | | | | | 23.8 | n 59-65 i |

**Tabelle 16**

| Mischung | Bsp.49 | Etgly | Hexgly | Vicoxet | Vicoxbu | Vicoxhe | Vimeox | Phasenverhalten |
|---|---|---|---|---|---|---|---|---|
| 126 | 82.8 | 17.2 | | | | | | n 75-81 i |
| 127 | 77.3 | | 22.7 | | | | | n 67-73 i |
| 128 | 80.0 | | | 20.0 | | | | n 109-111 i |
| 129 | 75.0 | | | 25.0 | | | | n 91-93 i |
| 130 | 59.3 | | | 40.7 | | | | n 97-100 i |
| 131 | 57.4 | | | 42.6 | | | | n 69-72 i |
| 132 | 47.7 | | | 52.3 | | | | n 92-95 i |
| 133 | 43.6 | | | 56.4 | | | | n 56-59 i |
| 134 | 80.8 | | | | 19.2 | | | n 101-103 i |
| 135 | 59.3 | | | | 40.7 | | | n 79-82 i |
| 136 | 38.0 | | | | 62.0 | | | n 56-58 i |
| 137 | 79.5 | | | | | 20.5 | | n 99-101 i |
| 138 | 59.9 | | | | | 40.1 | | n 68-71 i |
| 139 | 89.4 | | | | | | 10.6 | n 112-115 i |
| 140 | 76.0 | | | | | | 24.0 | n 108-119 i |
| 141 | 58.4 | | | | | | 41.6 | cr 57 n 105-117 i |

**Tabelle 17**

| Mischung | Bsp.49 | Mevimeox | Vicame | Vicaet | Vicabu | Vinon | Phasenverhalten |
|---|---|---|---|---|---|---|---|
| 142 | 89.0 | 11.0 | | | | | n 111-124 i |
| 143 | 75.1 | 24.9 | | | | | n 103-115 i |
| 144 | 57.3 | 42.7 | | | | | cr 55 n 91-102 i |
| 145 | 89.0 | | 11.0 | | | | n 115-133 i |
| 146 | 75.3 | | 24.7 | | | | n 119-132 i |
| 147 | 57.6 | | 42.4 | | | | n 118-131 i |
| 148 | 88.6 | | | 11.4 | | | n 120-130 i |
| 149 | 74.5 | | | 25.5 | | | n 123-131 i |
| 150 | 56.5 | | | 43.5 | | | n 130-135 i |
| 151 | 88.7 | | | | 11.3 | | n 114-126 i |
| 152 | 74.6 | | | | 25.4 | | n 110-121 i |
| 153 | 56.6 | | | | 43.4 | | cr 58 n 111-119 i |
| 154 | 86.5 | | | | | 13.5 | n 82-106 i |

## Patentansprüche

1. Polymerisierbare flüssigkristalline Verbindungen der allgemeinen Formel I
Z¹-Y¹-A¹-Y³-M-Y⁴-A²-Y²-Z² I,
in der die Variablen folgende Bedeutung haben:
Z¹, Z² Reste mit reaktiven Gruppen, über die eine Polymerisation herbeigeführt werden kann,
Y¹ - Y⁴ chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y³ und Y⁴ -O-CO-O-, -O-CO-NR-, -NR-CO-O oder -NR-CO-NR- bedeutet,
A¹, A² Spacer mit 2 bis 30 C-Atomen, in welchen die Kohlenstoffkette durch Sauerstoff in Etherfunktion, Schwefel in Thioetherfunktion oder durch nichtbenachbarte Imino- oder C₁-C₄-Alkyliminogruppen unterbrochen sein kann,
M eine mesogene Gruppe,
R C₁-C₄-Alkyl.

2. Polymerisierbare flüssigkristalline Verbindungen nach Anspruch 1, in denen mindestens eine der Gruppen Y³ und Y⁴ -O-CO-O- bedeutet.

3. Polymerisierbare flüssigkristalline Verbindungen nach Anspruch 1 oder 2, in denen die mesogene Gruppe M für eine Gruppe der Formel Ia
-(-T-Y⁵-)ᵣ-T- Ia
steht, in der die Variablen folgende Bedeutung haben:
T zweiwertige gesättigte oder ungesättigte iso- oder heterocyclische Reste,
Y⁵ Reste der Definition von Y¹ - Y⁴ oder -O-CH₂-, -CH₂-O-, -CH=N-, -N=CH- oder -N=N-,
r 0, 1, 2 oder 3,
wobei die Reste T und Y⁵ im Falle r > 0 gleich oder verschieden sein können.

4. Polymerisierbare flüssigkristalline Verbindungen nach den Ansprüchen 1 bis 3, in denen die mesogene Gruppe M eine Gruppe der folgenden Formeln 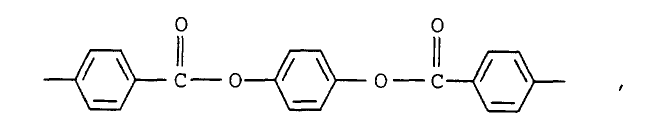 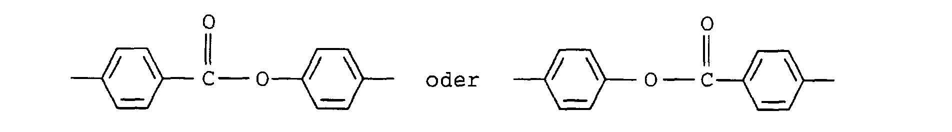 bedeutet, wobei jeder Ring bis zu drei gleiche oder verschiedene Substituenten aus der folgenden Gruppe tragen kann:
C₁-C₂₀-Alkyl, C₁-C₂₀-Alkoxy, C₁-C₂₀-Alkoxycarbonyl, C₁-C₂₀-Monoalkylaminocarbonyl, C₁-C₂₀-Alkylcarbonyl, C₁-C₂₀-Alkylcarbonyloxy, C₁-C₂₀-Alkylcarbonylamino, Formyl, Halogen, Cyan, Hydroxy und Nitro.

5. Polymerisierbare flüssigkristalline Verbindungen nach den Ansprüchen 1 bis 4, in denen die Restepaare Z¹ und Z², Y¹ und Y², Y³ und Y⁴ sowie A¹ und A² jeweils gleich sind.

6. Flüssigkristallzusammensetzungen, enthaltend
a) mindestens eine Verbindung gemäß den Ansprüchen 1 bis 5 ggf. in Mischung mit
b) einer oder mehreren Verbindungen der allgemeinen Formel II
Z³-Y⁶-A³-Y⁷-M-Y⁸-P¹ II,
in der die Variablen folgende Bedeutung haben:
Z³ Reste mit reaktiven Gruppen, über die eine Polymerisation herbeigeführt werden kann,
Y⁶ - Y⁸ chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y⁷ und Y⁸ -O-CO-O-, -O-CO-NR-, -NR-CO-O oder -NR-CO-NR- bedeutet,
A³ Spacer mit 2 bis 30 C-Atomen, in welchen die Kohlenstoffkette durch Sauerstoff in Etherfunktion, Schwefel in Thioetherfunktion oder durch nichtbenachbarte Imino- oder C₁-C₄-Alkyliminogruppen unterbrochen sein kann,
P¹ Reste, ausgewählt aus der Gruppe Wasserstoff, C₁-C₃₀-Alkyl, C₁-C₃₀-Acyl, C₃-C₈-Cycloalkyl ggf. substituiert durch ein bis drei C₁-C₆-Alkyl und wobei die Kohlenstoffkette der Alkyl-, Acyl- und Cycloalkylreste durch Sauerstoff in Etherfunktion, Schwefel in Tioetherfunktion oder durch nichtbenachbarte Imino- oder C₁-C₄-Alkyliminogruppen unterbrochen sein kann,
und/oder
c) einer oder mehreren Verbindungen der allgemeinen Formel III
P²-Y⁹-M-Y¹⁰-P³ III,
in der die Variablen folgende Bedeutung haben:
P²,P³ Reste, ausgewählt aus der Gruppe Wasserstoff, C₁-C₃₀-Alkyl, C₁-C₃₀-Acyl, C₃-C₈-Cycloalkyl ggf. substituiert durch ein bis drei C₁-C₆-Alkyl und wobei die Kohlenstoffkette der Alkyl-, Acylund Cycloalkylreste durch Sauerstoff in Etherfunktion, Schwefel in Tioetherfunktion oder durch nichtbenachbarte Imino- oder C₁-C₄-Alkyliminogruppen unterbrochen sein kann,
Y⁹, Y¹⁰ chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y⁹ und Y¹⁰ -O-CO-O-, -O-CO-NR-, -NR-CO-O oder -NR-CO-NR- bedeutet,
M eine mesogene Gruppe,
wobei die mesogenen Gruppen M der Formeln I, II und III gleich oder verschieden voneinander sein können.

7. Flüssigkristalline Verbindungen der Formel II
Z³-Y⁶-A³-Y⁷-M-Y⁸-P¹ II,
in der die Variablen folgende Bedeutung haben:
Z³ Reste mit reaktiven Gruppen, über die eine Polymerisation herbeigeführt werden kann,
Y⁶ Sauerstoff, Schwefel, -O-CO-, -CO-O-, -O-CO-O-, -CO-NR-, -NR-CO-, -O-CO-NR-, -NR-CO-O- oder -NR- CO-NR-,
Y⁷, Y⁸ chemische Einfachbindung, Sauerstoff, Schwefel, -O-CO-, -CO-O-, -O-CO-O-, -CO-NR-, -NR-CO-, -O-CO-NR-, -NR-CO-O- oder -NR-CO-NR-, wobei mindestens eine der Gruppen Y⁷ und Y⁸ -O-CO-O-, -O-CO-NR-, -NR-CO-O oder -NR-CO-NR- bedeutet,
A³ Spacer mit 2 bis 30 C-Atomen, in welchen die Kohlenstoffkette durch Sauerstoff in Etherfunktion, Schwefel in Thioetherfunktion oder durch nichtbenachbarte Imino- oder C₁-C₄-Alkyliminogruppen unterbrochen sein kann,
P¹ Reste, ausgewählt aus der Gruppe Wasserstoff, C₁-C₃₀-Alkyl, C₁-C₃₀-Acyl, C₃-C₈-Cycloalkyl ggf. substituiert durch ein bis drei C₁-C₆-Alkyl und wobei die Kohlenstoffkette der Alkyl-, Acyl- und Cycloalkylreste durch Sauerstoff in Etherfunktion, Schwefel in Tioetherfunktion oder durch nichtbenachbarte Imino- oder C₁-C₄-Alkyliminogruppen unterbrochen sein kann.

8. Flüssigkristallzusammensetzung, enthaltend eine oder mehrere der Verbindungen der Formeln I, II und III gemäß den Ansprüchen 1 bis 6 und eine oder mehrere chirale Verbindungen.

9. Flüssigkristallzusammensetzung gemäß Anspruch 6, enthaltend die Komponenten a), b) und c) in einem molaren Anteil an der Gesamtmenge der besagten Komponenten von
a) 1 bis 98 mol-%,
b) 1 bis 98 mol-%,
c) 0,01 bis 90 mol-%,
mit der Maßgabe, daß die Summe der mol-% der Komponenten 100 mol-% ergibt.

10. Flüssigkristallzusammensetzung gemäß Anspruch 8, enthaltend die Komponenten a), b) und c) in einem Anteil von 60 bis 99,999 Gew.-% und die chirale Verbindung oder die chiralen Verbindungen in einem Anteil von 0,001 bis 40 Gew.-%, jeweils bezogen auf die Gesamtmenge der Komponenten a), b) und c), sowie der chiralen Verbindungen oder den chiralen Verbindungen, mit der Maßgabe, daß die Summe der Gew.-% der Verbindungen 100 Gew.-% ergibt.

11. Flüssigkristallzusammensetzung, enthaltend 10 bis 100 Gew.-% der polymerisierbaren flüssigkristallinen Verbindungen I, I und II und/oder III gemäß den Ansprüchen 1 bis 7, 0 bis 90 Gew.-% weiterer Monomerer und 0 bis 50 Gew.-% einer oder mehrerer chiraler Verbindungen, jeweils bezogen auf das Gesamtgewicht der Flüssigkristallzusammensetzung.

12. Verfahren zur Herstellung von polymerisierbaren flüssigkristallinen Verbindungen der Formel I aus Anspruch 1, in welchen beide Variablen Y³ und Y⁴ zugleich -O-CO-O- sind, oder Mischungen aus diesen Verbindungen, dadurch gekennzeichnet, daß man ein oder mehrere Chlorformiate der Formel IVa
Z¹-Y¹-A¹-O-CO-Cl IVa
mit einem oder mehreren Mesogendiolen der Formel V
HO-M-OH V
zur symmetrischen Verbindung der Formel Ia
Z¹-Y¹-A¹-O-CO-O-M-O-CO-O-A¹-Y¹-Z¹ Ia
oder in einem ersten Schritt ein Chlorformiat der Formel IVa sowie in einem zweiten Schritt ein Chlorformiat der Formel IVb
Z²-Y²-A²-O-CO-Cl IVb
mit einem Mesogendiol der Formel V zur unsymmetrischen Verbindung Ib
Z¹-Y¹-A¹-O-CO-O-M-O-CO-O-A²-Y²-Z² Ib
umsetzt.

13. Verfahren zur Herstellung von flüssigkristallinen Verbindungen der Formel II aus Anspruch 6 bzw. 7, in welchen beide Variablen Y⁷ und Y⁸ zugleich -O-CO-O- sind, oder Mischungen aus diesen Verbindungen, dadurch gekennzeichnet, daß man in einem ersten Schritt ein Chlorformiat der Formel IVc
Z³-Y⁶-A³-O-CO-Cl IVc
bzw. ein Chlorformiat der Formel VI
P¹-O-CO-Cl VI
und in einem zweiten Schritt ein Chlorformiat der Formel VI bzw. ein Chlorformiat der Formel IVc mit einem oder mehreren Mesogendiolen der Formel V
HO-M-OH V
umsetzt.

14. Verfahren nach den Ansprüchen 9 bis 13, dadurch gekennzeichnet, daß man die Umsetzung in Gegenwart einer organischen und/oder anorganischen Base durchführt.

15. Verfahren zur Erzeugung von Beschichtungen mit flüssigkristallinem Ordnungszustand, dadurch gekennzeichnet, daß man eine oder mehrere flüssigkristalline Verbindungen gemäß den Ansprüchen 1 bis 5 oder Flüssigkristallzusammensetzungen gemäß Anspruch 6 und gewünschtenfalls weitere polymerisierbare Verbindungen und chirale Verbindungen auf ein Substrat aufbringt, in an sich bekannter Weise eine flüssigkristalline Orientierung herbeiführt und dann die auf das Substrat aufgebrachten Verbindungen polymerisiert.

16. Beschichtete Gegenstände, erhältlich nach dem Verfahren gemäß Anspruch 15.

17. Verwendung der polymerisierbaren flüssigkristallinen Verbindungen gemäß den Ansprüchen 1 bis 5 sowie der Flüssigkristallzusammensetzungen gemäß den Ansprüchen 6 und 8 bis 11 für die Herstellung optischer Anzeigegeräte.

18. Verwendung von Flüssigkristallzusammensetzungen gemäß den Ansprüchen 8, 10 und 11 als cholesterisch flüssigkristalline Farbmittel.

19. Cholesterisch flüssigkristalline Farbmittel, enthaltend Flüssigkristallzusammensetzungen gemäß den Ansprüchen 8, 10 und 11.

20. Pigmente, dadurch erhältlich, daß man Flüssigkristallzusammensetzungen gemäß den Ansprüchen 8, 10 und 11 der Polymerisation unterwirft und die polymere Masse danach auf Pigment-Teilchengröße zerkleinert.

## Claims

1. A polymerizable liquid-crystalline compound of the formula I
Z¹-Y¹-A¹-Y³-M-Y⁴-A²-Y²-Z² I
where
Z¹ and Z² are radicals containing reactive groups via which polymerization can be effected,
Y¹ - Y⁴ are a single chemical bond, oxygen, sulfur, -O-CO-, -CO-O-, -O-CO-O-, -CO-NR-, -NR-CO-, -O-CO-NR-, -NR-CO-O- or -NR-CO-NR-, where at least one of the groups Y³ and Y⁴ is -O-CO-O-, -O-CO-NR-, -NR-CO-O- or -NR-CO-NR-,
A¹ and A² are spacers having 2 to 30 carbon atoms in which the carbon chain may be interrupted by ether oxygen, thioether sulfur or by nonadjacent imino or C₁-C₄-alkylimino groups,
M is a mesogenic group,
R is C₁-C₄-alkyl.

2. A polymerizable liquid-crystalline compound as claimed in claim 1, where at least one of the groups Y³ and Y⁴ is -O-CO-O-.

3. A polymerizable liquid-crystalline compound as claimed in claim 1 or 2, where the mesogenic group M is a group of the formula Ia
-(-T-Y⁵-)ᵣ-T- Ia
where
T is a divalent saturated or unsaturated iso- or heterocyclic radical,
Y⁵ is a radical as defined for Y¹ - Y⁴ or is -O-CH₂-, -CH₂-O-, -CH=N-, -N=CH- or -N=N-,
r is 0, 1, 2 or 3,
where the radicals T and Y⁵, in the case where r is > 0, may be identical or different.

4. A polymerizable liquid-crystalline compound as claimed in any of claims 1 to 3, where the mesogenic group M is a group of the following formulae 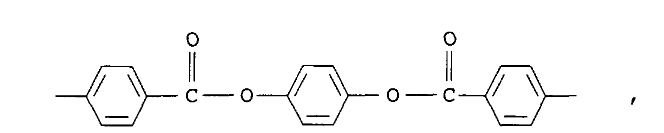 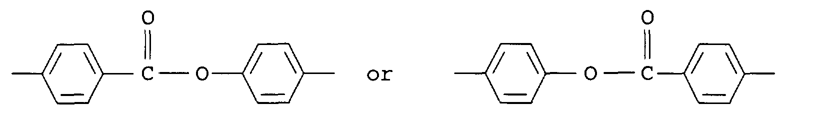 where each ring can carry up to three identical or different substituents from the following group:
C₁-C₂₀-alkyl, C₁-C₂₀-alkoxy, C₁-C₂₀-alkoxycarbonyl, C₁-C₂₀-monoalkylaminocarbonyl, C₁-C₂₀-alkylcarbonyl, C₁-C₂₀-alkylcarbonyloxy, C₁-C₂₀-alkylcarbonylamino, formyl, halogen, cyano, hydroxyl and nitro.

5. A polymerizable liquid-crystalline compound as claimed in any of claims 1 to 4, where the radical pairs Z¹ and Z², Y¹ and Y², Y³ and Y⁴, and A¹ and A² are in each case identical.

6. A liquid-crystal composition comprising
a) at least one compound as claimed in any of claims 1 to 5, which may be mixed with
b) one or more compounds of the formula II
Z³-Y⁶-A³-Y⁷-M-Y⁸-P¹ II,
where
Z³ are radicals containing reactive groups via which polymerization can be effected,
Y⁶ - Y⁸ are a single chemical bond, oxygen, sulfur, -O-CO-, -CO-O-, -O-CO-O-, -CO-NR-, -NR-CO-, -O-CO-NR-, -NR-CO-O- or -NR-CO-NR-, where at least one of the groups Y⁷ and Y⁸ is -O-CO-O-, -O-CO-NR-, -NR-CO-O or -NR-CO-NR-,
A³ is a spacer having 2 to 30 carbon atoms in which the carbon chain may be interrupted by ether oxygen, thioether sulfur or by nonadjacent imino or C₁-C₄-alkylimino groups,
P¹ are radicals selected from the group of hydrogen, C₁-C₃₀-alkyl, C₁-C₃₀-acyl, C₃-C₈-cycloalkyl unsubstituted or substituted by one to three C₁-C₆-alkyl and where the carbon chain of the alkyl, acyl and cycloalkyl radicals may be interrupted by ether oxygen, thioether sulfur or by nonadjacent imino or C₁-C₄-alkylimino groups,
and/or
c) one or more compounds of the formula III
P²-Y⁹-M-Y¹⁰-P³ III,
where
P²,P³ are radicals selected from the group of hydrogen, C₁-C₃₀-alkyl, C₁-C₃₀-acyl, C₃-C₈-cycloalkyl unsubstituted or substituted by one to three C₁-C₆-alkyl, and where the carbon chain of the alkyl, acyl and cycloalkyl radicals may be interrupted by ether oxygen, thioether sulfur or by nonadjacent imino or C₁-C₄-alkylimino groups,
Y⁹, Y¹⁰ are a single chemical bond, oxygen, sulfur, -O-CO-, -CO-O-, -O-CO-O-, -CO-NR-, -NR-CO-, -O-CO-NR-, -NR-CO-O- or -NR-CO-NR-, where at least one of the groups Y⁹ and Y¹⁰ is -O-CO-O-, -O-CO-NR-, -NR-CO-O or -NR-CO-NR-,
M is a mesogenic group,
where the mesogenic groups M of the formulae I, II and III can be identical to or different from one another.

7. A liquid-crystalline compound of the formula II
Z³-Y⁶-A³-Y⁷-M-Y⁸-P¹ II,
where
Z³ are radicals containing reactive groups via which polymerization can be effected,
Y⁶ is oxygen, sulfur, -O-CO-, -CO-O-, -O-CO-O-, -CO-NR-, -NR-CO-, -O-CO-NR-, -NR-CO-O- or -NR-CO-NR-,
Y⁷,Y⁸ are a single chemical bond, oxygen, sulfur,-O-CO-, -CO-O-, -O-CO-O-, -CO-NR-, -NR-CO-, -O-CO-NR-, -NR-CO-O- or -NR-CO-NR-, where at least one of the groups Y⁷ and Y⁸ is -O-CO-O-, -O-CO-NR-, -NR-CO-O or -NR-CO-NR-,
A³ is a spacer having 2 to 30 carbon atoms in which the carbon chain may be interrupted by ether oxygen, thioether sulfur or by nonadjacent imino or C₁-C₄-alkylimino groups,
P¹ are radicals selected from the group of hydrogen, C₁-C₃₀-alkyl, C₁-C₃₀-acyl, C₃-C₈-cycloalkyl unsubstituted or substituted by one to three C₁-C₆-alkyl and where the carbon chain of the alkyl, acyl and cycloalkyl radicals may be interrupted by ether oxygen, thioether sulfur or by nonadjacent imino or C₁-C₄-alkylimino groups.

8. A liquid-crystal composition comprising one or more compounds of the formulae I, II and III as claimed in any of claims 1 to 6 and one or more chiral compounds.

9. A liquid-crystal composition as claimed in claim 6, comprising components a), b) and c) in a molar proportion based on the total amount of said components of
a) 1-98 mol%.
b) 1-98 mol%,
c) 0.01-90 mol%,
with the proviso that the total of the mol% of the components is 100 mol%.

10. A liquid-crystal composition as claimed in claim 8, comprising components a), b) and c) in a proportion of from 60 to 99.999% by weight and the chiral compound or chiral compounds in a proportion of from 0.001 to 40% by weight, in each case based on the total amount of components a), b) and c), and of the chiral compound or chiral compounds, with the proviso that the total of the percent by weight of the compounds is 100% by weight.

11. A liquid-crystal composition comprising 10 to 100% by weight of the polymerizable liquid-crystalline compounds I, I and II and/or III as claimed in any of claims 1 to 7, 0 to 90% by weight of other monomers and 0 to 50% by weight of one or more chiral compounds, in each case based on the total weight of the liquid-crystal composition.

12. A process for preparing polymerizable liquid-crystalline compounds of the formula I from claim 1, where the two variables Y³ and Y⁴ are both -O-CO-O-, or mixtures of these compounds, which comprises reacting one or more chloroformates of the formula IVa
Z¹-Y¹-A¹-O-CO-Cl IVa
with one or more mesogen diols of the formula V
HO-M-OH V
to give the symmetrical compound of the formula Ia
Z¹-Y¹-A¹-O-CO-O-M-O-CO-O-A¹-Y¹-Z¹ Ia
or reacting in a first step a chloroformate of the formula IVa and in a second step a chloroformate of the formula IVb
Z²-Y²-A²-O-CO-Cl IVb
with a mesogen diol of the formula V to give the asymmetrical compound Ib
Z¹-Y¹-A¹-O-CO-O-M-O-CO-O-A²-Y²-Z² Ib.

13. A process for preparing liquid-crystalline compounds of the formula II from claim 6 or 7, where the two variables Y⁷ and Y⁸ are both -O-CO-O-, or mixtures of these compounds, which comprises reacting in a first step a chloroformate of the formula IVc
Z³-Y⁶-A³-O-CO-Cl IVc
or a chloroformate of the formula VI
P¹-O-CO-Cl VI
and in a second step a chloroformate of the formula VI or a chloroformate of the formula IVc with one or more mesogen diols of the formula V
HO-M-OH V.

14. A process as claimed in any of claims 9 to 13, wherein the reaction is carried out in the presence of an organic and/or inorganic base.

15. A process for producing coatings having a liquid-crystalline ordered state, which comprises applying one or more liquid-crystalline compounds as claimed in any of claims 1 to 5 or liquid-crystal compositions as claimed in claim 6 and, if desired, further polymerizable compounds and chiral compounds to a substrate, effecting a liquid-crystalline alignment in a manner known per se, and then polymerizing the compounds applied to the substrate.

16. A coated article obtainable by a process as claimed in claim 15.

17. The use of a polymerizable liquid-crystalline compound as claimed in any of claims 1 to 5 and of the liquid-crystal composition as claimed in any of claims 6 and 8 to 11 for the production of optical display devices.

18. The use of a liquid-crystal composition as claimed in any of claims 8, 10 and 11 as a cholesteric liquid-crystalline colorant.

19. A cholesteric liquid-crystalline colorant comprising a liquid-crystal composition as claimed in any of claims 8, 10 and 11.

20. A pigment obtainable by subjecting a liquid-crystal composition as claimed in any of claims 8, 10 and 11 to polymerization and then comminuting the polymeric material to the particle size of pigments.

## Revendications

1. Composés de cristaux liquides polymérisables de formule générale I
Z¹-Y¹-A¹-Y³-M-Y⁴-A²-Y²-Z² I,
dans laquelle les variables prennent la signification suivante :
Z¹, Z² représentent des résidus ayant des groupes réactifs, par le biais desquels une polymérisation peut être provoquée,
Y¹ à Y⁴ représentent une liaison simple chimique, un atome d'oxygène, de soufre, -O-CO-, -CO-O-, -O-CO-O-, -CO-NR-,-NR-CO-, -O-CO-NR-, -NR-CO-O ou -NR-CO-NR-, où au moins un des groupes Y³ et Y⁴ représente -O-CO-O-, -O-CO-NR, -NR-CO-O ou -NR-CO-NR-,
A¹, A² représentent des intercalaires ayant 2 à 30 atomes de C, dans lesquels la chaîne carbonée peut être interrompue par un atome d'oxygène dans la fonction éther, par un atome de soufre dans la fonction thioéther ou par des groupes imino ou alkylimino en C₁ à C₄ non vicinaux,
M représente un groupe mésogène,
R représente un groupe alkyle en C₁ à C₄.

2. Composés de cristaux liquides polymérisables selon la revendication 1, dans lesquels au moins l'un des groupes Y³ et Y⁴ représente -O-CO-O-.

3. Composés de cristaux liquides polymérisables selon la revendication 1 ou 2, dans lesquels le groupe mésogène M représente un groupe de formule Ia
-(-T-Y⁵-)ᵣ-T- Ia
dans laquelle, les variables prennent la signification suivante :
T représente des résidus bivalents, saturés ou insaturés, iso- ou hétéro-cycliques,
Y⁵ représente un résidu selon la définition de Y¹ à Y⁴ ou bien -O-CH₂-, -CH₂-O-, -CH=N-, -N=CH- ou -N=N-,
r vaut 0, 1, 2 ou 3,
les résidus T et Y⁵ pouvant être identiques ou différents dans le cas où r > 0.

4. Composés de cristaux liquides polymérisables selon les revendications 1 à 3, dans lesquelles le groupe mésogène représente un groupe des formules suivantes 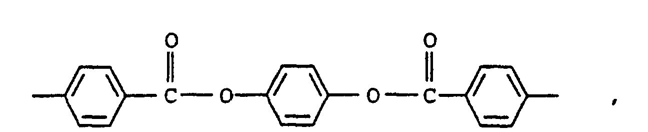 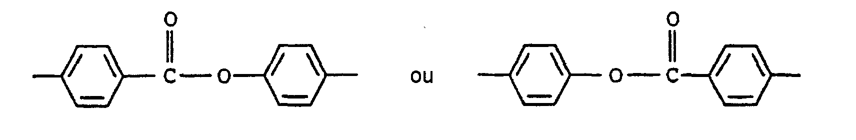 où chaque noyau peut porter jusqu'à trois substituants identiques ou différents choisis dans le groupe suivant : les groupes alkyle en C₁ à C₂₀, alcoxy en C₁ à C₂₀, (alcoxy en C₁ à C₂₀)carbonyle, (monoalkyle en C₁ à C₂₀)aminocarbonyle, (alkyle en C₁ à C₂₀)carbonyle, (alkyle en C₁ à C₂₀)carbonyloxy, (alkyle en C₁ à C₂₀)carbonylamino, formyle, halogéno, cyano, hydroxy et nitro.

5. Composés de cristaux liquides polymérisables selon les revendications 1 à 4, dans lesquelles les paires de résidus Z¹ et Z², Y¹ et Y², Y³ et Y⁴, ainsi que A¹ et A², sont respectivement identiques.

6. Composition de cristaux liquides, contenant
a) au moins un composé selon les revendications 1 à 5 éventuellement en mélange avec
b) un ou plusieurs composés de formule générale II
Z³-Y⁶-A³-Y⁷-M-Y⁸-P¹ II,
dans laquelle les variables prennent la signification suivante :
Z³ représente des résidus ayant des groupes réactifs par le biais desquels une polymérisation peut être provoquée,
Y⁶ à Y⁸ représentent une liaison simple chimique, un atome d'oxygène, de soufre, -O-CO-, -CO-O-, -O-CO-O-, -CO-NR-,-NR-CO-, -O-CO-NR-, -NR-CO-O ou -NR-CO-NR-, où au moins un des groupes Y⁷ et Y⁸ représente -O-CO-O-, -O-CO-NR, -NR-CO-O ou -NR-CO-NR-,
A³ représente des intercalaires ayant 2 à 30 atomes de C, dans lesquels la chaîne carbonée peut être interrompue par un atome d'oxygène dans la fonction éther, par un atome de soufre dans la fonction thioéther ou par des groupes imino ou alkylimino en C₁ à C₄ non vicinaux,
P¹ représente des résidus choisis dans le groupe formé par l'atome d'hydrogène, un groupe alkyle en C₁ à C₃₀, acyle en C₁ à C₃₀, cycloalkyle en C₃ à C₈, éventuellement substitué par un à trois groupes alkyle en C₁ à C₆, et dans lequel la chaîne carbonée des résidus alkyle, acyle et cycloalkyle peut être interrompue par un atome d'oxygène dans la fonction éther, un atome de soufre dans la fonction thioéther ou par des groupes imino ou alkylimino en C₁ à C₄, non vicinaux,
et/ou
c) un ou plusieurs composés de formule générale III
P²-Y⁹-M-Y¹⁰-P³ III,
dans laquelle les variables prennent la signification suivante :
P², P³ représentent des résidus choisis dans le groupe formé par l'atome d'hydrogène, un groupe alkyle en C₁ à C₃₀, acyle en C₁ à C₃₀, cycloalkyle en C₃ à C₈, éventuellement substitué par un à trois groupes alkyles en C₁ à C₆, et dans lesquels la chaîne carbonée des résidus alkyles, acyles et cycloalkyles peut être interrompue par un atome d'oxygène dans la fonction éther, un atome de soufre dans la fonction thioéther ou par des groupes imino ou alkylimino en C₁ à C₄, non vicinaux,
Y⁹, Y¹⁰ représentent une liaison simple chimique, un atome d'oxygène, de soufre, -O-CO-, -CO-O-, -O-CO-O-, -CO-NR-, -NR-CO-, -O-CO-NR-, -NR-CO-O ou -NR-CO-NR-, où au moins un des groupes Y⁹ et Y¹⁰ représente -O-CO-O-, -O-CO-NR, -NR-CO-O ou -NR-CO-NR-,
M représente un groupe mésogène,
où les groupes mésogènes M des formules I, II et III peuvent être identiques ou différents les uns des autres.

7. Composés de cristaux liquides de formule II
Z³-Y⁶-A³-Y⁷-M-Y⁸-P¹ II,
dans laquelle les variables prennent la signification suivante :
Z³ représente des résidus ayant des groupes réactifs, par le biais desquels une polymérisation peut être provoquée,
Y⁶ représente un atome d'oxygène, de soufre, -O-CO-,-CO-O-, -O-CO-O-, -CO-NR-, -NR-CO-, -O-CO-NR-, -NR-CO-O ou-NR-CO-NR-,
Y⁷, Y⁸ représentent une liaison simple chimique, un atome d'oxygène, de soufre, -O-CO-, -CO-O-, -O-CO-O-, -CO-NR-, -NR-CO-, -O-CO-NR-, -NR-CO-O ou -NR-CO-NR-, où au moins un des groupes Y⁷ et Y⁸ représente -O-CO-O-, -O-CO-NR, -NR-CO-O ou -NR-CO-NR-,
A³ représente des intercalaires ayant 2 à 30 atomes de C, dans lesquels la chaîne carbonée peut être interrompue par un atome d'oxygène dans la fonction éther, un atome de soufre dans la fonction thioéther ou par des groupes imino ou alkylimino en C₁ à C₄ non vicinaux,
P¹ représente des résidus choisis dans le groupe formé par l'atome d'hydrogène, un groupe alkyle en C₁ à C₃₀, acyle en C₁ à C₃₀, cycloalkyle en C₃ à C₈, éventuellement substitué par un à trois groupes alkyle en C₁ à C₆, et où la chaîne carbonée des résidus alkyle, acyle et cycloalkyle peut être interrompue par un atome d'oxygène dans la fonction éther, un atome de soufre dans la fonction thioéther ou par des groupes imino ou alkylimino en C₁ à C₄, non vicinaux.

8. Composition de cristaux liquides contenant un ou plusieurs des composés des formules I, II et III selon les revendications 1 à 6 et un ou plusieurs composés chiraux.

9. Composition de cristaux liquides selon la revendication 6, contenant les composants a), b), et c) dans une proportion molaire par rapport à la quantité totale des composants cités, de
a) 1 à 98% en moles,
b) 1 à 98% en moles,
c) 0,01 à 90% en moles,
avec la condition que la somme des pourcentages molaires des composants donne 100% en moles.

10. Composition de cristaux liquides selon la revendication 8, contenant les composants a), b), et c) dans une proportion de 60 à 99,999% en poids et le composé chiral ou les composés chiraux en une proportion de 0,001 à 40% en pois, à chaque fois par rapport à la quantité totale des composants a), b) et c), ainsi que le ou les composés chiraux, avec la condition que la somme des pourcentages pondéraux des composés donne 100 % en poids.

11. Composition de cristaux liquides contenant 10 à 100% en poids des composés de cristaux liquides polymérisables I, I et II et/ou III selon les revendications 1 à 7, 0 à 90% en poids d'autres monomères, et 0 à 50% en poids d'un ou plusieurs composés chiraux, respectivement par rapport au poids total de la composition de cristaux liquides.

12. Procédé de préparation de composés de cristaux liquides polymérisables de formule I selon la revendication 1, dans lequel les deux variables Y³ et Y⁴ représentent toutes deux -O-CO-O-, ou de mélanges de ces composés, caractérisés en ce que l'on met un ou plusieurs chloroformiates de formule IVa
Z¹-Y¹-A¹-O-CO-Cl IVa
à réagir avec un ou plusieurs diols mésogènes de formule V
HO-M-OH V
pour obtenir le composé symétrique de formule Ia
Z¹-Y¹-A¹-O-CO-O-M-O-CO-O-A¹-Y¹-Z¹ Ia,
ou bien dans une première étape, on met un chloroformiate de formule IVa ainsi que dans une deuxième étape, un chloroformiate de formule IVb
Z²-Y²-A²-O-CO-Cl IVb,
à réagir avec un diol mésogène de formule V pour obtenir le composé asymétrique Ib
Z¹-Y¹-A¹-O-CO-O-M-O-CO-O-A²-Y²-Z² Ib,

13. Procédé de préparation de composés de cristaux liquides de formule II selon la revendication 6 ou 7, dans lequel les deux variables Y⁷ et Y⁸ sont toutes deux -O-CO-O-, ou de mélanges de ces composés, caractérisé en ce que l'on met dans une première étape, un chloroformiate de formule IVc
Z³-Y⁶-A³-O-CO-Cl IVc
ou bien un chloroformiate de formule VI
P¹-O-CO-Cl VI
et dans une deuxième étape un chloroformiate de formule VI ou un chloroformiate de formule IVc, à réagir avec un ou plusieurs diols mésogènes de formule V
HO-M-OH V

14. Procédé selon les revendications 9 à 13, caractérisé en ce que l'on réalise la réaction en présence d'une base organique et/ou inorganique.

15. Procédé de production de revêtements ayant un état ordonné de cristaux liquides, caractérisé en ce que l'on applique sur un substrat, un ou plusieurs composés de cristaux liquides selon les revendications 1 à 5 ou des compositions de cristaux liquides selon la revendication 6, et éventuellement d'autres composés polymérisables et composés chiraux, l'on provoque d'une manière connue une orientation de cristaux liquides puis on polymérise les composés appliqués sur le substrat.

16. Objets revêtus, pouvant être obtenus d'après le procédé selon la revendication 15.

17. Utilisation des composés de cristaux liquides polymérisables selon les revendications 1 à 5 ainsi que des compositions de cristaux liquides selon les revendications 6 et 8 à 11 pour la préparation de dispositifs d'affichage optiques.

18. Utilisation des compositions de cristaux liquides selon les revendications 8, 10 et 11 en tant que colorants de cristaux liquides cholestériques.

19. Colorants de cristaux liquides cholestériques, contenant des compositions de cristaux liquides selon les revendications 8, 10 et 11.

20. Pigments pouvant être obtenus en soumettant des compositions de cristaux liquides selon les revendications 8, 10 et 11, à la polymérisation et en broyant ensuite la masse polymère jusqu'à obtenir une taille de particule de pigment.
